# EUROPEAN PATENT APPLICATION

(11) **EP 3 754 022 A1**
(43) Date of publication of application: **23.12.2020**
(21) Application number: 19754563.5
(22) Date of filing: 15.02.2019
(51) Int. Cl.: C12N 15/12, A61K 35/15, A61K 35/17, A61K 35/76, A61K 38/08, A61K 38/10, A61K 38/16, A61K 48/00, A61P 1/04, A61P 11/00, A61P 35/00, C07K 7/08, C07K 14/47, C07K 19/00, C12N 5/0783, C12N 5/10, C12N 15/63, C07K 14/74, C12P 21/02

(54) **CANCER ANTIGEN PEPTIDE**

(30) Priority: 15.02.2018 JP 2018024972
(71) Applicant: National University Corporation Asahikawa Medical University, Asahikawa-shi, Hokkaido 078-8510 (JP)
(72) Inventor: OHKURI, Takayuki, Asahikawa-shi, Hokkaido 078-8510 (JP); KOSAKA, Akemi, Asahikawa-shi, Hokkaido 078-8510 (JP); KOBAYASHI, Hiroya, Asahikawa-shi, Hokkaido 078-8510 (JP)
(74) Representative: Hoffmann Eitle
(86) International application number: PCT/JP2019/005623
(87) International publication number: WO 2019/160099

(57) **Abstract**

The disclosure provides a peptide consisting of 10 to 45 amino acids and comprising the amino acid sequence of KILQQSRIVQX, wherein X is absent or S; an amino acid sequence of 10 or more contiguous amino acids in the amino acid sequence of DVQKIVESQINFHGKKLKLGPAIRKQNLCAYHVQPRPL (SEQ ID NO: 16); or the amino acid sequence of QNLNHYIQVLENLVRSVPS (SEQ ID NO: 9); or a peptide having an amino acid sequence that is different from the amino acid sequence of the former peptide in that 1 to 3 amino acids are substituted, deleted or added and being capable of activating a helper T-cell, as well as products relating to the peptide such as an polynucleotide. The disclosure also provides use of the peptide and the products as a medicament or a composition for activating a helper T-cell.

## Description

### TECHNICAL FIELD

This application claims the benefit of priority of Japanese Patent Application No. 2018-024972, the entire contents of which are incorporated herein by reference.

The disclosure relates to a novel cancer antigen peptide.

### BACKGROUND

The immune system has a mechanism for eliminating cancer cells by recognizing highly immunogenic cancer antigen proteins that are expressed when normal cells become cancerous. Since recent studies have revealed that effective activation of the immune system is extremely important for controlling cancer growth, as demonstrated by the remarkable treatment effect of immune checkpoint inhibitors such as anti-PD-1 antibodies, cancer vaccine therapy is drawing attention.

Most of the antigen molecules selected as targets in conventional cancer vaccine therapy were molecules that are rarely or moderately expressed in normal tissues but highly expressed only in "grown" cancer tissues. However, cancer cells have various immunoediting mechanisms to escape from the immune system. When normal cells become cancerous, the cells are growing with reducing the expression of molecules that are easily targeted by the immune system (Non-Patent Literature 1). In other words, it is suggested that antigens expressed in "grown" cancer tissues are hardly targeted by the immune system, and thus cancer tissues do not have to reduce the expression of such antigens during the process of "growing". Accordingly, the target antigens that have been selected in conventional cancer vaccine therapy are possibly antigens insufficient to activate the immune system.

One of the reported mechanisms by which cancer cells reduce the expression of antigens unfavorable to themselves is suppressing the expression of the immunogenic cancer antigen genes by methylation of the promoter regions (Non-Patent Literature 2). This suggests cancer cells that have escaped from the immune surveillance to form cancer tissues have hidden immunogenic cancer antigen proteins unfavorable to themselves by methylating their promoter regions.

In order to establish more effective cancer vaccine therapy, it is demanded to identify immunogenic cancer antigens "unfavorable to cancer cells", which are downregulated by the cancer cells during the process of cancer growth, i.e., stealth cancer antigens.

### REFERENCES

### NON-PATENT LITERATURE

[Non-Patent Literature 1] Schreiber RD et al. Science 2011, Mar 25;331(6024):1565-70
[Non-Patent Literature 2] DuPage et al. Nature 2012 Feb 8;482(7385):405-9

### SUMMARY

An object of the disclosure is to provide a novel cancer antigen peptide and use thereof.

The inventors have found some stealth cancer antigens by using a DNA methyltransferase inhibitor and identified their partial peptides capable of activating helper T-cells.

Accordingly, an aspect of the disclosure provides a peptide consisting of 10 to 45 amino acids and comprising the amino acid sequence of KILQQSRIVQX, wherein X is absent or S;
an amino acid sequence of 10 or more contiguous amino acids in the amino acid sequence of DVQKIVESQINFHGKKLKLGPAIRKQNLCAYHVQPRPL (SEQ ID NO: 16); or the amino acid sequence of QNLNHYIQVLENLVRSVPS (SEQ ID NO: 9), or
a peptide having an amino acid sequence that is different from the amino acid sequence of the former peptide in that 1 to 3 amino acids are substituted, deleted or added and being capable of activating a helper T-cell.

An aspect of the disclosure provides a nucleic acid which encodes the peptide.

An aspect of the disclosure provides an expression vector comprising the nucleic acid.

An aspect of the disclosure provides an HLA multimer comprising the peptide and an HLA class II molecule.

An aspect of the disclosure provides an antigen-presenting cell presenting a complex of the peptide and an HLA class II molecule.

An aspect of the disclosure provides a helper T-cell capable of recognizing a complex of the peptide and an HLA class II molecule.

An aspect of the disclosure provides a pharmaceutical composition comprising the peptide, the nucleic acid, the expression vector, the antigen-presenting cell, or the helper T-cell.

An aspect of the disclosure provides a composition for activating a helper T-cell comprising the peptide, the nucleic acid, the expression vector, or the antigen-presenting cell.

The peptide disclosed herein, which can activate helper T-cells specific for a stealth cancer antigen, should be useful for treatment or prevention of a cancer that can express the stealth cancer antigen.

### BRIEF DESCRIPTION OF DRAWINGS

Fig. 1 shows the gene expression levels of SYCP3 in the cells of cancer cell lines EBC1 and Lu65 which were treated with 5-AZA.
Fig. 2 shows the gene expression levels of SYCP3 in the cells of cancer cell lines HT-29 and SAS which were treated with 5-AZA.
Fig. 3 shows the gene expression levels of SPESP1 in the cells of cancer cell lines SW839, 5637, LC2/Ad, and EBC1 which were treated with 5-AZA.
Fig. 4 shows the gene expression levels of SPESP1 in the cells of cancer cell lines HT-29, Lu65, and SAS which were treated with 5-AZA.
Fig. 5 shows the gene expression levels of DAZL1 in the cells of cancer cell line WEHI-3 which were treated with 5-AZA.
Fig. 6 shows the gene expression levels of SYCP3 in the tumor tissues collected from immunodeficient mice to which cells of a colorectal cancer cell line were injected and 5-AZA was administered.
Fig. 7 shows the gene expression levels of SPESP1 in the tumor tissues collected from immunodeficient mice to which cells of a lung cancer cell line were injected and 5-AZA was administered.
Fig. 8 shows the reactivity of CD4-positive T-cells toward the partial SYCP3-A peptides.
Fig. 9 shows the ability of DAZL-1C peptide to activate helpler T-cells in mice.
Fig. 10 shows the reactivity of the SYCP3-A-specific Th cells toward the SYCP3-A stimulation.
Fig. 11 shows the reactivity of the SYCP3-A-specific Th cells toward the partial SYCP3-A peptides.
Fig. 12 shows the reactivity of the SYCP3-A-specific Th cells toward the partial SYCP3-A peptides.
Fig. 13 shows the reactivity of the SPESP1-B-specific Th cells toward the SPESP1-B stimulation.
Fig. 14 shows the reactivity of the DAZL1-specific Th cells toward the partial DAZL1 peptides.
Fig. 15 shows the reactivity of the SYCP3-A-specific Th cells toward the DR53-positive cancer cells which were treated with 5-AZA.
Fig. 16 shows tumor growth suppressing effect of the combination of 5-AZA and the SYCP3-specific human Th cells.

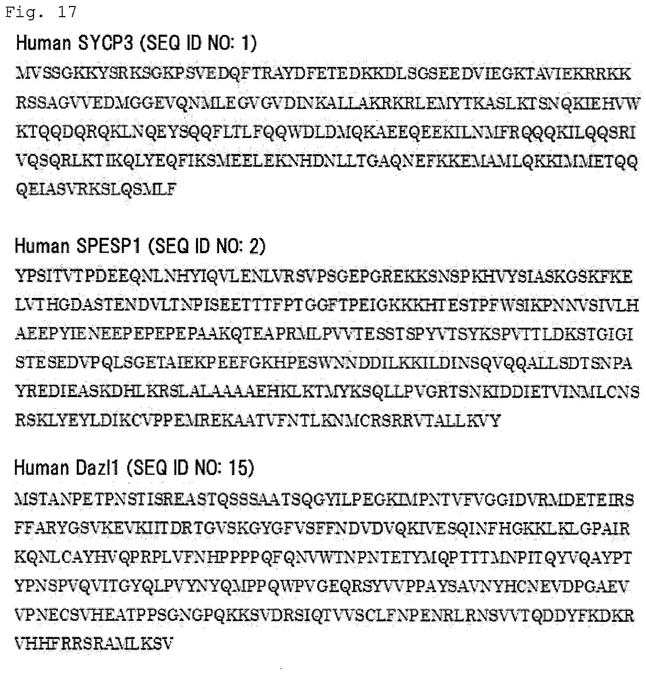

Fig. 17 shows the amino acid sequences of human SYCP3 (SEQ ID NO: 1), human SPESP1 (SEQ ID NO: 2), and human DAZL1 (SEQ ID NO: 15).

### DETAILED DESCRIPTION

Unless otherwise defined, the terms used herein are read as generally understood by a skilled person in the technical fields such as organic chemistry, medical sciences, pharmaceutical sciences, molecular biology, and microbiology. Several terms used herein are defined as described below. The definitions herein take precedence over the general understanding.

The term "stealth cancer antigen" as used herein means a protein whose expression is suppressed through methylation of the promoter region of the gene by the immunoediting system of cancer cells. Examples of the stealth cancer antigens include SYCP3 (synaptonemal complex protein 3), SPESP1 (sperm equatorial segment protein 1), and DAZL1 (deleted in azoospermia-like 1). SYCP3 is an important component of the synaptonemal complex which is involved in chromosome pairing, recombination, and segregation in meiosis. Mutations in SYCP3 are associated with azoospermia and infertility. Human SYCP3 may have the amino acid sequence of SEQ ID NO: 1. SPESP1 is a human alloantigen involved in spermegg binding and fusion. Human SPESP1 may have the amino acid sequence of SEQ ID NO: 2. DAZL1 is a member of the DAZ family and is an RNA binding protein expressed in prenatal and postnatal germ cells of both sexes. Human DAZL1 may have the amino acid sequence of SEQ ID NO: 15.

The term "helper peptide" as used herein means a peptide derived from a cancer antigen protein and capable of activating a helper T-cell.

Examples of the helper peptides derived from SYCP3 include peptides comprising an amino acid sequence selected from KILQQSRIVQ (SEQ ID NO: 3) and KILQQSRIVQS (SEQ ID NO: 4). In an embodiment, the helper peptide comprises an amino acid sequence selected from SEQ ID NOs: 3 and 4 and consists of contiguous amino acids in the amino acid sequence of SEQ ID NO: 1. In an embodiment, the helper peptide consists of an amino acid sequence selected from SEQ ID NOs: 3 and 4. In an embodiment, the helper peptide comprises an amino acid sequence selected from KILQQSRIVQSQ (SEQ ID NO: 5), QKILQQSRIVQS (SEQ ID NO: 6), QQKILQQSRIVQ (SEQ ID NO: 7), and QQQKILQQSRIVQSQRLKT (SEQ ID NO: 8), or consists of an amino acid sequence selected from SEQ ID NOs: 5 to 8, especially consists of an amino acid sequence selected from SEQ ID NOs: 5 and 6.

Examples of the peptides comprising an amino acid sequence selected from SEQ ID NOs: 5 to 8 include peptides comprising an amino acid sequence selected from RQQQKILQQSRIVQSQRLKT (SEQ ID NO: 22), LNMFRQQQKILQQSRIVQSQRLKT (SEQ ID NO: 23), QQQKILQQSRIVQSQRLKTI (SEQ ID NO: 24), and QQQKILQQSRIVQSQRLKTIKQLY (SEQ ID NO: 25), and the peptides consisting of an amino acid sequence selected from SEQ ID NOs: 22 to 25.

Further examples of the helper peptides derived from SYCP3 include peptides comprising an amino acid sequence selected from KILQQSRVVQ (SEQ ID NO: 26) and KILQQSRVVQS (SEQ ID NO: 27) and the peptides consisting of an amino acid sequence selected from SEQ ID NOs: 26 and 27. In an embodiment, the helper peptide comprises an amino acid sequence selected from KILQQSRVVQSQ (SEQ ID NO: 28), QKILQQSRVVQS (SEQ ID NO: 29), QQKILQQSRVVQ (SEQ ID NO: 30), QQQKILQQSRVVQSQRLKT (SEQ ID NO: 31), RQQQKILQQSRVVQSQRLKT (SEQ ID NO: 32), LNMFRQQQKILQQSRVVQSQRLKT (SEQ ID NO: 33), QQQKILQQSRVVQSQRLKTI (SEQ ID NO: 34), and QQQKILQQSRVVQSQRLKTIKQLY (SEQ ID NO: 35), or consists of an amino acid sequence selected from SEQ ID NOs: 28 to 35.

The amino acid sequences of SEQ ID NOs: 3 and 4 may be comprehensively represented herein by Sequence (I): KILQQSRIVQX, wherein X is absent or S. The amino acid sequences of SEQ ID NOs: 26 and 27 may be comprehensively represented herein by Sequence (I'): KILQQSRVVQX, wherein X is absent or S.

Examples of the helper peptides derived from SPESP1 include peptides comprising the amino acid sequence of QNLNHYIQVLENLVRSVPS (SEQ ID NO: 9). In an embodiment, the helper peptide comprises the amino acid sequence of SEQ ID NO: 9 and consists of contiguous amino acids in the amino acid sequence of SEQ ID NO: 2. In an embodiment, the helper peptide consists of the amino acid sequence of SEQ ID NO: 9.

Examples of the helper peptides derived from DAZL1 include peptides comprising an amino acid sequence of 10 or more contiguous amino acids in the amino acid sequence of DVQKIVESQINFHGKKLKLGPAIRKQNLCAYHVQPRPL (SEQ ID NO: 16). In an embodiment, the helper peptide comprises an amino acid sequence of 20 or more contiguous amino acids in the amino acid sequence of SEQ ID NO: 16. In an embodiment, the helper peptide comprises an amino acid sequence of 10 or more or 20 or more contiguous amino acids in the amino acid sequence of SEQ ID NO: 16 and consists of contiguous amino acids in the amino acid sequence of SEQ ID NO: 15. In an embodiment, the helper peptide comprises an amino acid sequence selected from SEQ ID NO: 16, DVQKIVESQINFHGKKLKLG (SEQ ID NO: 17), INFHGKKLKLGPAIRKQNLC (SEQ ID NO: 18), LGPAIRKQNLCAYHVQPRPL (SEQ ID NO: 19), DVQKIVESQINFHGKKLKLGPAIRKQNLC (SEQ ID NO: 20), and INFHGKKLKLGPAIRKQNLCAYHVQPRPL (SEQ ID NO: 21), or consists of an amino acid sequence selected from SEQ ID NOs: 16 to 21.

The helper peptide disclosed herein has a length allowing its binding to an MHC class II molecule, for example, a length of 10 to 45, 10 to 40, 10 to 35, 10 to 30, or 10 to 25 amino acids. For example, the helper peptide may consist of 10 to 18, 10 to 19, 10 to 20, 11 to 18, 11 to 19, 11 to 20, 12 to 18, 12 to 19, or 12 to 20 amino acids. It is generally thought that an antigen peptide which binds to an MHC class II molecule may be longer, because the peptide has an MHC class II molecule binding motif consisting of about 9 amino acids, the motif binds to a peptide-binding groove of the MHC class II molecule, and thus the both ends of the peptide may stick out from the groove. For example, the helper peptide may consist of 10 to 45, 15 to 40, or 20 to 38 amino acids. However, a longer peptide is generally cleaved by a peptidase to a length of 13 to 17 amino acids (Immunobiology, 5th Edt., 116-117, Garland Publishing (2001)).

The helper peptide may consist of an amino acid sequence that is different from any one of the amino acid sequences mentioned above in that one or more amino acids are substituted, deleted or added. Any number of amino acid residues at any positions may be substituted, deleted, or added, as long as the ability to activate helper T-cells is retained. For example, the helper peptide may consist of an amino acid sequence that is different from any one of the amino acid sequences mentioned above in that 1 to 9, 1 to 5, 1 to 4, 1 to 3, or 1 to 2, e.g., 1, amino acid(s) is/are substituted, deleted or added. In virtue of the nature of the helper peptide as described above any number of amino acids, e.g., 1 to 20, 1 to 15, or 1 to 10 amino acids, may be added at the N- or C-terminus.

The substitution may occur between any amino acids. Conservative amino acid substitution is preferred. The term "conservative amino acid substitution" means substitution of an amino acid residue to another amino acid residue having a side chain of the similar property. Amino acid residues are classified into some families on the basis of the side chains. Examples of the side chains include a basic side chain (e.g., lysin, arginine, and histidine), an acidic side chain (e.g., asparatic acid and glutamic acid), an uncharged polar side chain (e.g., asparagine, glutamine, serine, threonine, tyrosine, and cysteine), a nonpolar side chain (e.g., glycine, alanine, valine, leucine, isoleucine, proline, phenylalanine, methionine, and tryptophane), a β-branched side chain (e.g., threonine, valine, and isoleucine), an aliphatic side chain (e.g., glycine, alanine, valine, leucine, isoleucine, serine, and threonine), an aromatic side chain (e.g., tyrosine, phenylalanine, and tryptophane), an amide side chain (e.g., asparagine and glutamine), and a sulfur-containing side chain (e.g., cysteine and methionine). The conservative amino acid substitution is preferably a substitution between amino acid residues within the same family. Examples of the conservative amino acid substitutions include substitutions of a glutamic acid residue to an aspartic acid residue, a phenylalanine residue to a tyrosine residue, a leucine residue to an isoleucine residue, an isoleucine residue to a valine residue, an alanine residue to a serine residue, and a histidine residue to an arginine residue.

One or more amino acids may be substituted so that the sequence feature (motif) common in the antigen peptides capable of binding to a desired MHC class II molecule is retained. In general, an antigen peptide gets into a peptide-binding groove of an MHC class II molecule and is fixed. The fixation is achieved by binding of the side chains of the amino acid residues of the peptide to the peptide-binding groove and binding of the main chain of the peptide to the side chains of the amino acid residues which are preserved in the peptide-binding grooves of all MHC class II molecules. The motif of amino acid residues which binds to the peptide-binding groove of a desired MHC class II molecule can be deduced by analyzing the pattern of the amino acid residues commonly found in the peptides capable of binding to the MHC class II molecule. Amino acid polymorphism is observed among the amino acid residues constituting small and large pockets of the peptide-binding groove. For each MHC class II molecule derived from each allele, each amino acid motif can be deduced.

In an embodiment, a helper peptide derived from SYCP3 or SPESP1 may activate helper T-cells through binding to HLA-DR, especially HLA-DR53, without limitation. In an embodiment, a helper peptide derived from DAZL1 may activate helper T-cells through binding to HLA-DR, especially HLA-DR4, 8, 9, 15, or 53, without limitation. In an embodiment, a helper peptide which is derived from DAZL1 and comprises the amino acid sequence of SEQ ID NO: 17 may activate helper T-cells through binding to HLA-DR4, 9, or 53, without limitation. In an embodiment, a helper peptide which is derived from DAZL1 and comprises the amino acid sequence of SEQ ID NO: 18 may activate helper T-cells through binding to HLA-DR15, without limitation. In an embodiment, a helper peptide which is derived from DAZL1 and comprises the amino acid sequence of SEQ ID NO: 19 may activate helper T-cells through binding to HLA-DR8, without limitation.

One or more amino acid residues of the peptide may be modified by any known method. Examples of the modifications include esterification, alkylation, acylation (e.g., acetylation), halogenation, and phosphorylation on functional groups in side chains of amino acid residues, the amino group of the amino acid at the N-terminus, or the carboxyl group of the amino acid at the C-terminus. In an embodiment, the N-terminus of the helper peptide is acetylated. It is also possible to add one or more substances, e.g., amino acids, peptides, and analogs thereof, to the N-terminus and/or C-terminus of the helper peptide. For example, a histidine tag may be added, or a fusion protein may be formed together with a protein such as thioredoxin. Alternatively, a detectable label may be bound to the helper peptide. When such substance is bound to the helper peptide, the substance may be processed, for example, with a biologic enzyme or through intracellular processing, to produce the helper peptide. Such substance may regulate the solubility of the helper peptide, improve the stability of the peptide such as protease resistance, allow specific delivery of the helper peptide to a desired tissue or organ, or enhance the uptake of the helper peptide by antigen presenting cells. Such substance may be a substance to increase the ability of the peptide to induce CTL, for example, another peptide that activates helper T-cells.

The peptide can be synthesized using a method usually used in the art or a modified one. Such synthesis methods are disclosed, for example, in Peptide Synthesis, Interscience, New York, 1966; The Proteins, Vol 2, Academic Press Inc., New York, 1976; Peptide Synthesis, Maruzen Co., Ltd., 1975; Basis and Experiments of Peptide Synthesis, Maruzen Co., Ltd., 1985; and Development of Medicines (continuation), Vol. 14, Peptide Synthesis, Hirokawa Shoten Co., 1991. The peptide also can be prepared using a genetic engineering technique on the basis of the information of the nucleotide sequence encoding the peptide. Such genetic engineering techniques are well known to those skilled in the art. Such techniques can be conducted, for example, according to the methods described in the literatures (e.g., Molecular Cloning, T. Maniatis et al., CSH Laboratory (1983) and DNA Cloning, DM. Glover, IRL PRESS (1985)).

In general, a helper T-cell is activated when a TCR-CD3 complex on the T-cell surface recognizes an antigen peptide complexed with an MHC class II molecule on the surface of an antigen presenting cell, and an integrin on the T-cell surface is stimulated by an integrin ligand on the surface of the antigen presenting cell. In the disclosure the activation of helper T-cells includes induction of helper T-cells, enhancement of proliferation of .helper T-cells, and induction of cytokine production of helper T-cells.

The ability of a variant peptide to activate helper T-cells may be determined by synthesizing the peptide and testing whether the peptide could activate helper T-cells. For such test, the method described in Hassane M. Zarour et al., Cancer Research 62, 213-218, January 1, 2002, the method described in the Examples, or the following method may be used.

Dendritic cells (adherent cells) are prepared by collecting peripheral blood mononuclear cells (PBMCs) from a human subject and removing non-adherent cells. Helper T-cells (CD4-positive T-cells) are separately prepared from the same subject, for example, by density gradient centrifugation with Ficoll-Paque or magnetic cell sorting. The dendritic cells are cultured with a candidate peptide, mixed with the helper T-cells and cultured. The helper T-cells are then recovered and stimulated several times in a similar manner with the dendritic cells cultured with the candidate peptide. The activation (induction) of the helper T-cells can be confirmed, for example, by determining (1) the proliferative activity of the helper T-cells or (2) the cytokine-producing-activity of the helper T-cells. The proliferative activity (1) can be determined, for example, by measuring the amount of [³H] -thymidine incorporated into the helper T-cells. The cytokine-producing activity (2) can be determined, for example, by measuring the amount of cytokines such as IFN-γ produced by the activated helper T-cells by a method such as enzyme enzyme-linked immunosorbent assay (ELISA).

In an aspect, the disclosure provides a polynucleotide encoding the helper peptide. The polynucleotide may be in the form of any nucleic acid, such as DNA or RNA. The polynucleotide can be easily prepared on the basis of the information about the amino acid sequence of the peptide or the polynucleotide sequence of the DNA encoding it. Specifically, the polynucleotide may be prepared by a usual method of DNA synthesis or amplification by PCR.

The polynucleotide provided herein may encompass a polynucleotide that hybridizes to the complementary sequence of the polynucleotide encoding the helper peptide under a stringent condition and that encodes a peptide which activates helper T-cells. Regarding "hybridize under a stringent condition", the hybridization can be carried out according to any conventional method, for example, those described in Molecular Cloning, T. Maniatis et al., CSH Laboratory (1983). The stringent condition may be a condition where the hybridization is conducted in a solution containing 6 x SSC (10 x SSC is a solution containing 1.5 M NaCl and 0.15 M trisodium citrate) and 50% formamide at 45°C, followed by washing in 2 x SSC at 50°C (Molecular Biology, John Wiley & Sons, N. Y. (1989), 6.3.1-6.3.6).

A recombinant expression vector for expressing the helper peptide can be constructed by incorporating the polynucleotide as prepared above into an expression vector. Any expression vector may be used depending on the host and the purpose. The examples of the vectors include plasmids, phage vectors, and virus vectors. For example, when the host is Escherichia coli, examples of the vectors include plasmid vectors, such as pUC118, pUC119, pBR322, and pCR3, and phage vectors, such as λZAPII and λgt11. When the host is yeast, examples of the vectors include pYES2 and pYEUra3. When the host is an insect cell, examples of the vectors include pAcSGHisNT-A. When the host is an animal cell, examples of the vectors include plasmid vectors, such as pKCR, pCDM8, pGL2, pcDNA3.1, pRc/RSV, and pRc/CMV, and virus vectors, such as retrovirus vectors, adenovirus vectors, and adeno-associated virus vectors.

The expression vector may optionally contain a factor such as a promoter capable of inducing expression, a gene encoding a signal sequence, a marker gene for selection, and a terminator.

Furthermore, the expression vector may contain an additional sequence for generating a fusion protein with a moiety to facilitate the isolation and purification, such as thioredoxin, His tag, or GST (glutathione S-transferase). Examples of such vectors include GST fusion protein vectors (e.g., pGEX4T), vectors containing a tag sequence such as Myc or His (e.g., pcDNA3.1/Myc-His), and vectors capable of expressing a fusion protein with thioredoxin and His tag (e.g., pET32a), containing an appropriate promoter (e.g., lac, tac, trc, trp, CMV, or SV40 early promoter) that is functional in the host cells.

Transformed cells containing the expression vector can be prepared by transforming the host cells with the vector obtained as described. Examples of the hosts include Escherichia coli, yeast, insect cells, and animal cells. Examples of the Escherichia coli strains include the strains of E. coli K-12 such as HB101, C600, JM109, DH5α, and AD494 (DE3). Examples of the yeast species include Saccharomyces cerevisiae. Examples of the animal cells include L929, BALB/c3T3 cells, C127 cells, CHO cells, COS cells, Vero cells, and Hela cells. Examples of the insect cells include sf9.

The expression vector may be introduced into the host cells by using a conventional method suitable for the host cells, for example, a calcium phosphate method, a DEAE-dextran method, an electroporation method, and a method using a lipid for gene transfer (e.g., Lipofectamine, Lipofectin; Gibco-BRL). Following the introduction, the cells may be cultured in a conventional medium containing the selection marker to obtain the transformed cells containing the expression vector.

The helper peptide can be produced by culturing the transformed cells under an appropriate condition. The produced peptide may be further isolated and purified according to a standard biochemical purification procedure. Examples of the purification procedures include salting out, ion exchange chromatography, absorption chromatography, affinity chromatography, and gel filtration chromatography. When the helper peptide is expressed as a fusion peptide with thioredoxin, His tag, or GST, as described, the peptide can be isolated and purified by an appropriate purification procedure using the characteristics of the fusion protein or tag.

In an aspect, the disclosure provides an antibody which specifically binds to the helper peptide. The antibody may be a polyclonal or monoclonal antibody. The antibody can be prepared according to a conventional method (Current protocols in Molecular Biology edit. Ausubel et al. (1987) Publish. John Wiley and Sons., Antibodies; A Laboratory Manual, Lane, H, D. et al., ed., Cold Spring Harber Laboratory Press, New York 1989). A polyclonal antibody can be obtained by immunizing a non-human animal such as a rabbit using the peptide as an antigen, and recovering the antibody from the serum of the immunized animal in a conventional manner. A monoclonal antibody can be obtained by immunizing a non-human animal such as a mouse with the peptide, subjecting the resultant splenocytes to cell fusion with myeloma cells to generate hybridoma cells, and recovering the monoclonal antibody from the hybridoma cells. The immunological response may be enhanced with an adjuvant suitable for the host animal.

The antibody, which can recognize the helper peptide and neutralize its activity, may be used, for example, for affinity chromatography or an immunological diagnostic method. The immunological diagnostic method may be carried out, for example, by using immunoblotting, radioimmunoassay (RIA), enzyme-linked immunosorbent assay (ELISA), or a fluorescent or luminescent assay. Such immunological diagnostic method is effective in the diagnosis of cancers in which expression of SYCP3, SPESP1, or DAZL1 is induced by suppression of DNA methylation, e.g., hematological diseases such as leukemia, malignant melanoma, breast cancer, head and neck tumor, urinary tumor, esophageal cancer, liver cancer, lung cancer, or colon cancer.

In an aspect, the disclosure provides an HLA multimer comprising the helper peptide and an MHC class II molecule. The HLA multimer as used herein means a multimer of HLA monomers, which is obtained by making two or more HLA monomers bind to each other by a known method. The HLA monomer is a complex in which the peptide is associated with an HLA protein. A helper peptide derived from SYCP3 or SPESP1 may form a complex with HLA-DR53, without limitation. A helper peptide derived from DAZL1 may form a complex with HLA-DR4, 8, 9, 15, or 53, without limitation. The multimer may be fluorescently labeled so that the helper T-cells bound by the multimer can be selected or detected easily by a known detecting means, such as flow cytometry or fluorescence microscopy. The HLA multimer may be a tetramer, a pentamer, or a dendrimer, for example, an HLA tetramer prepared by making biotinylated HLA monomers bind to an avidin molecule. As HLA tetramers containing various antigen peptides are commercially available, the HLA tetramer containing the helper peptide may be easily prepared in a similar manner (Science 279: 2103-2106(1998), Science 274: 94-96 (1996)).

In an aspect, the disclosure provides an antigen presenting cell presenting a complex of the peptide and an HLA class II molecule. In an embodiment, a helper peptide derived from SYCP3 or SPESP1 may form a complex with HLA-DR53, without limitation. In an embodiment, a helper peptide derived from DAZL1 may form a complex with HLA-DR4, 8, 9, 15, or 53, without limitation. The antigen presenting cell may be derived from a cell capable of presenting a complex of the peptide and an HLA class II molecule to a helper T-cell, such as a peripheral blood mononuclear cell or a dendritic cell.

The antigen presenting cell may be prepared by adding the peptide to a cell having an antigen presenting ability by a technique known to those skilled in the art. The peptide may be added directly as the peptide itself or indirectly through the polynucleotide, the vector, or the transformed cell as described. For example, the peptide may be added by allowing a cell having an antigen presenting ability to contact with the peptide or introducing the polynucleotide or the expression vector into such a cell (Cancer Immunol. Immunother. 46:82, 1998; J. Immunol., 158: p1796, 1997; Cancer Res., 59: p1184, 1999; Cancer Res., 56: p5672, 1996; J.Immunol., 161: p5607, 1998; J. Exp. Med., 184: p465, 1996). The cell having an antigen presenting ability as used herein is a cell expressing an MHC class II molecule on the cell surface, including a peripheral blood mononuclear cell and a dendritic cell. The antigen presentation may be confirmed by determining the activity of helper T-cells, as shown in the Examples below. The activity of helper T-cells may be confirmed, for example, by production of a cytokine such as interferon-γ. The antigen presenting cell may be used as an active ingredient in cell therapy (e.g., dendritic cell therapy).

In a further aspect, the disclosure provides the helper peptide, the polynucleotide, the vector, the multimer, or a cell comprising any one of these components for preparing an antigen presenting cell. In a further aspect, the disclosure provides use of the helper peptide, the polynucleotide, the vector, the multimer, or a cell comprising any one of these components for preparing an antigen presenting cell.

The antigen presenting cell as described can activate a helper T-cell that recognizes a complex of the helper peptide and an HLA class II molecule. Accordingly, in an aspect the disclosure provides a composition for activating a helper T-cell comprising the helper peptide, the polynucleotide, the vector, the multimer, a cell comprising any one of these components, or the antigen presenting cell.

An activated helper T-cell may activate the immune system by enhancing induction, proliferation, and activation of B cells and cytotoxic T-cells. Accordingly, the composition for activating a helper T-cell or the activated helper T-cell may be used for enhancing induction, proliferation and activation of B cells and/or cytotoxic T-cells, or for activating the immune system thereby.

The composition for activating a helper T-cell may contain a component other than the active ingredient, such as a carrier, an excipient, or an additive. The composition may be used *in vitro* or *in vivo.* The *in vivo* use of the composition may be in accordance with the use of the pharmaceutical composition described below. The usage of the composition may be selected properly depending on factors such as the desired degree of the helper T-cell activation and the condition of the antigen presenting cell. For example, the composition may be administered to a subject, for example with intradermal administration, subcutaneous administration, intramuscular administration, intravenous administration, transnasal administration, or oral administration, or may be added to a culture medium, without limitation. Usage of the composition, for example the amount of the active ingredient contained in the composition, the type of the.composition, or the frequency of use, may be selected properly depending on factors such as the desired degree of the helper T-cell activation and the condition of the antigen presenting cell.

In an aspect, the disclosure provides the helper peptide, the polynucleotide, the vector, the multimer, a cell comprising any one of these components, or the antigen presenting cell for activating a helper T-cell. In an aspect, the disclosure provides use of the helper peptide, the polynucleotide, the vector, the multimer, a cell comprising any one of these components, or the antigen presenting cell for manufacturing a composition for activating a helper T-cell. In an aspect, the disclosure provides a method of activating a helper T-cell comprising administrating the helper peptide, the polynucleotide, the vector, the multimer, a cell comprising any one of these components, or the antigen presenting cell to a subject in need thereof. In an aspect, the disclosure provides a method of activating a helper T-cell comprising adding the helper peptide, the polynucleotide, the vector, the multimer, a cell comprising any one of these components, or the antigen presenting cell to a helper T-cell *in vitro.*

In an aspect, the disclosure provides a helper T-cell which recognizes a complex of the helper peptide with an MHC class II molecule. A helper peptide derived from SYCP3 or SPESP1 may form a complex with HLA-DR53, without limitation. A helper peptide derived from DAZL1 may form a complex with HLA-DR4, 8, 9, 15, or 53, without limitation. The helper T-cell can be easily prepared by those skilled in the art using a technique known in the art (Iwata, M. et al., Eur. J. Immunol, 26, 2081 (1996)).

In another aspect, the disclosure provides a pharmaceutical composition comprising the helper peptide, the polynucleotide, the vector, the multimer, a cell comprising any one of these components, the antigen presenting cell, or the helper T-cell as the active ingredient. The pharmaceutical composition may be used for treating or preventing a cancer or for assisting it. In an embodiment, the pharmaceutical composition is a cancer vaccine.

The pharmaceutical composition can treat or prevent a cancer in which expression of SYCP3, SPESP1, or DAZL1 is induced by suppression of DNA methylation. The cancer may be a solid tumor or a hematologic tumor, e.g., hematological diseases such as leukemia, malignant melanoma, breast cancer, head and neck tumor, urinary tumor, esophageal cancer, liver cancer, lung cancer, or colon cancer. In an embodiment, when the helper peptide is derived from SYCP3 or SPESP1, the pharmaceutical composition may be administered to a subject having HLA-DR53, without limitation. In an embodiment, when the helper peptide is derived from DAZL1, the pharmaceutical composition may be administered to a subject having HLA-DR4, 8, 9, 15, or 53, without limitation.

The pharmaceutical composition may contain one or more components other than the active ingredient, such as a carrier or an excipient. The administration method of the composition may be selected properly depending on factors such as the type of the disease, the state of the subject, and the target site. The administration method includes, but is not limited to, intradermal administration, subcutaneous administration, intramuscular administration, intravenous administration, transnasal administration, and oral administration. Details of the administration, such as the amount of the active ingredient contained in the pharmaceutical composition, the dosage form of the composition, and the administration frequency, may be selected properly depending on factors such as the type of the disease, the state of the subject, and the target site.

The pharmaceutical composition may contain or be used in combination with at least one additional active ingredient. Examples of the additional active ingredients include chemotherapeutic agents, such as antimetabolites, alkylating agents, anticancer antibiotics, antimicrotubule agents, platinum based drugs, topoisomerase inhibitors, molecular target drugs, cancer vaccines, immunomodulators, immune checkpoint inhibitors, and DNA methyltransferase inhibitors; and activators, proliferative agents, or inducers of helper T-cells or cytotoxic T-cells. Clinicians of ordinal skill can determine the additional active ingredient and the therapeutically effective amount thereof within their skill and judgment. The pharmaceutical composition may be used in parallel with, or before or after other therapy such as chemotherapy, radiation therapy, immunotherapy, hematopoietic stem cell transplantation, or surgery.

In an embodiment, the additional active ingredient is a DNA methyltransferase inhibitor. Examples of the DNA methyltransferase inhibitors include the drugs disclosed in J. Med. Chem. 2015, 58, 2569-2583, including decitabine, guadecitabine, azacitidine, zebularine, tetrahydrouridine, tetrahydrouridine, and derivatives thereof, especially decitabine and azacitidine.

When some ingredients are used "in combination", a dosage form containing all the ingredients may be administered, or a combination of dosage forms containing each ingredient, i.e., a kit, may be administered. Alternatively, the combination may be achieved by administering all the ingredients simultaneously, sequentially, or separately, i.e., one or more ingredients may be administered at later time points, as long as the ingredients are used for treating the same disease.

In an aspect, the disclosure provides a method of treating or preventing a cancer comprising administrating an effective amount of the helper peptide, the polynucleotide, the vector, the multimer, a cell comprising any one of these components, the antigen presenting cell, or the helper T-cell to a subject in need thereof.

In an aspect, the disclosure provides the helper peptide, the polynucleotide, the vector, the multimer, a cell comprising any one of these components, the antigen presenting cell, or the helper T-cell for use as a medicament, for example for use in treatment or prevention of a cancer.

In an aspect, the disclosure provides use of the helper peptide, the polynucleotide, the vector, the multimer, a cell comprising any one of these components, the antigen presenting cell, or the helper T-cell for manufacturing a medicament, for example a medicament for treating or preventing a cancer.

In another aspect, the disclosure provides a method for determining the presence or amount of helper T-cells specific for a stealth cancer antigen in a subject, comprising:
(a) stimulating a sample obtained from the subject with a helper peptide, and
(b) determining the amount of helper T-cells or the cytokines produced by the helper T-cells,
wherein the increase of the amount determined in step (b) indicates the presence or amount of the helper T-cells specific for the stealth cancer antigen.

Any sample may be used as long as it contains antigen presenting cells, such as peripheral blood mononuclear cells, invasive lymphocytes, tumor cells, cells in ascites fluid, cells in pleural effusion, cells in cerebrospinal fluid, bone marrow cells, and lymph node cells. The sample may be derived from a healthy donor or from a cancer patient. A sample derived from a healthy donor may be used for diagnosing whether the donor is actually affected by a cancer, or whether the donor has a predisposition of a cancer. A sample derived from a cancer patient may be used for diagnosing whether the immunotherapy using the stealth cancer antigen is effective in the patient. In an embodiment, the amount of helper T-cells specific for a helper peptide derived from SYCP3 or SPESP1 in an HLA-DR-positive subject, especially an HLA-DR53-positive subject, is determined, without limitation. In an embodiment, the amount of helper T-cells specific for a helper peptide derived from DAZL1 in an HLA-DR-positive subject, especially an HLA-DR4, 8, 9, 15, or 53-positive subject, is determined, without limitation. An obtained sample may be cultured before and/or after stimulation with a helper peptide, and the culture conditions may be determined properly by those skilled in the art. The stimulation of these cells with a helper peptide may be carried out using a known technique, and may be carried out either *in vitro* or *in vivo.* The amount of helper T-cells or the cytokines produced by the helper T-cells may be determined by a known method.

For example, the disclosure provides the following embodiments.
[1] A peptide consisting of 10 to 25 amino acids and comprising the amino acid sequence of KILQQSRIVQX, wherein X is absent or S, or QNLNHYIQVLENLVRSVPS (SEQ ID NO: 9).
[2] The peptide according to item 1, comprising the amino acid sequence of KILQQSRIVQX, wherein X is absent or S.
[3] The peptide according to item 1 or 2, consisting of contiguous amino acids in the amino acid sequence of SEQ ID NO: 1.
[4] The peptide according to any one of items 1 to 3, comprising an amino acid sequence selected from KILQQSRIVQ (SEQ ID NO: 3), KILQQSRIVQS (SEQ ID NO: 4), KILQQSRIVQSQ (SEQ ID NO: 5), QKILQQSRIVQS (SEQ ID NO: 6), QQKILQQSRIVQ (SEQ ID NO: 7), and QQQKILQQSRIVQSQRLKT (SEQ ID NO: 8).
[5] The peptide according to any one of items 1 to 4, consisting of an amino acid sequence selected from SEQ ID NOs: 3 to 8.
[6] The peptide according to any one of items 1 to 3, comprising an amino acid sequence selected from SEQ ID NOs: 3 and 4.
[7] The peptide according to any one of items 1 to 3 and 6, consisting of an amino acid sequence selected from SEQ ID NOs: 3 and 4.
[8] The peptide according to any one of items 1 to 3, comprising the amino acid sequence of SEQ ID NO: 3.
[9] The peptide according to any one of items 1 to 3 and 8, consisting of the amino acid sequence of SEQ ID NO: 3.
[10] The peptide according to any one of items 1 to 3, comprising the amino acid sequence of SEQ ID NO: 4.
[11] The peptide according to any one of items 1 to 3 and 10, consisting of the amino acid sequence of SEQ ID NO: 4.
[12] The peptide according to any one of items 1 to 3, comprising an amino acid sequence selected from SEQ ID NOs: 5 to 8.
[13] The peptide according to any one of items 1 to 3 and 12, consisting of an amino acid sequence selected from SEQ ID NOs: 5 to 8.
[14] The peptide according to any one of items 1 to 3, comprising an amino acid sequence selected from SEQ ID NOs: 5 to 7.
[15] The peptide according to any one of items 1 to 3 and 14, consisting of an amino acid sequence selected from SEQ ID NOs: 5 to 7.
[16] The peptide according to any one of items 1 to 3, comprising an amino acid sequence selected from SEQ ID NOs: 5 and 6.
[17] The peptide according to any one of items 1 to 3 and 16, consisting of an amino acid sequence selected from SEQ ID NOs: 5 and 6.
[18] The peptide according to any one of items 1 to 3, comprising the amino acid sequence of SEQ ID NO: 5.
[19] The peptide according to any one of items 1 to 3 and 18, consisting of the amino acid sequence of SEQ ID NO: 5.
[20] The peptide according to item 1, comprising the amino acid sequence of QNLNHYIQVLENLVRSVPS (SEQ ID NO: 9).
[21] The peptide according to item 1 or 20, consisting of contiguous amino acids in the amino acid sequence of SEQ ID NO: 2.
[22] The peptide according to item 1, 20, or 21, consisting of the amino acid sequence of SEQ ID NO: 9.
[23] A peptide consisting of 10 to 25 amino acids and comprising
   the amino acid sequence of KILQQSRIVQX, wherein X is absent or S, or QNLNHYIQVLENLVRSVPS (SEQ ID NO: 9),
   or
   a peptide having an amino acid sequence that is different from the amino acid sequence of the former peptide in that 1 to 3 amino acids are substituted, deleted or added and being capable of activating a helper T-cell.
[24] A nucleic acid which encodes the peptide according to any one of items 1 to 23.
[25] An expression vector comprising the nucleic acid according to item 24.
[26] A transformed cell comprising the expression vector according to item 25.
[27] An antibody which specifically binds to the peptide according to any one of items 1 to 23.
[28] An HLA multimer comprising the peptide according to any one of items 1 to 23 and an HLA class II molecule.
[29] An antigen-presenting cell presenting a complex of the peptide according to any one of items 1 to 23 and an HLA class II molecule.
[30] A helper T-cell capable of recognizing a complex of the peptide according to any one of items 1 to 23 and an HLA class II molecule.
[31] A pharmaceutical composition comprising the peptide according to any one of items 1 to 23, the nucleic acid according to item 24, the expression vector according to item 25, the HLA multimer according to item 28, the antigen-presenting cell according to item 29, or the helper T-cell according to item 30.
[32] The pharmaceutical composition according to item 31, for treating or preventing a cancer.
[33] The pharmaceutical composition according to item 31 or 32, which is a cancer vaccine.
[34] The pharmaceutical composition according to item 32 or 33, wherein the cancer is lung cancer or colon cancer.
[35] A composition for activating a helper T-cell comprising the peptide according to any one of items 1 to 23, the nucleic acid according to item 24, the expression vector according to item 25, the HLA multimer according to item 28, or the antigen-presenting cell according to item 29.

For example, the disclosure further provides the following embodiments.
[1] A peptide consisting of 10 to 45 amino acids and comprising the amino acid sequence of KILQQSRIVQX, wherein X is absent or S;
   an amino acid sequence of 10 or more contiguous amino acids in the amino acid sequence of DVQKIVESQINFHGKKLKLGPAIRKQNLCAYHVQPRPL (SEQ ID NO: 16); or the amino acid sequence of QNLNHYIQVLENLVRSVPS (SEQ ID NO: 9), or
   a peptide having an amino acid sequence that is different from the amino acid sequence of the former peptide in that 1 to 3 amino acids are substituted, deleted or added and being capable of activating a helper T-cell.
[2] The peptide according to item 1, which is
   a peptide consisting of 10 to 25 amino acids and comprising the amino acid sequence of KILQQSRIVQX, wherein X is absent or S,
   or
   a peptide having an amino acid sequence that is different from the amino acid sequence of the former peptide in that one amino acid is substituted, deleted or added and being capable of activating a helper T-cell.
[3] The peptide according to item 2, consisting of 10 to 25 amino acids and comprising the amino acid sequence of KILQQSRIVQX, wherein X is absent or S, or KILQQSRVVQX, wherein X is absent or S.
[4] The peptide according to item 2 or 3, comprising an amino acid sequence selected from SEQ ID NOs: 5 to 7 and 28 to 30.
[5] The peptide according to item 2 or 3, consisting of an amino acid sequence selected from SEQ ID NOs: 3 to 8 and 22 to 35.
[6] The peptide according to item 2, consisting of 10 to 25 amino acids and comprising the amino acid sequence of KILQQSRIVQX, wherein X is absent or S.
[7] The peptide according to item 6, consisting of contiguous amino acids in the amino acid sequence of SEQ ID NO: 1.
[8] The peptide according to item 6 or 7, comprising an amino acid sequence selected from SEQ ID NOs: 5 to 7.
[9] The peptide according to item 6 or 7, consisting of an amino acid sequence selected from SEQ ID NOs: 3 to 8 and 22 to 25.
[10] The peptide according to item 6, 7, or 9, consisting of an amino acid sequence selected from SEQ ID NOs: 3 to 8.
[11] The peptide according to item 1, which is
   a peptide consisting of 10 to 45 amino acids and comprising an amino acid sequence of 10 or more contiguous amino acids in the amino acid sequence of SEQ ID NO: 16, or
   a peptide having an amino acid sequence that is different from the amino acid sequence of the former peptide in that one amino acid is substituted, deleted or added and being capable of activating a helper T-cell.
[12] The peptide according to item 11, consisting of 10 to 45 amino acids and comprising an amino acid sequence of 10 or more contiguous amino acids in the amino acid sequence of SEQ ID NO: 16.
[13] The peptide according to item 11 or 12, consisting of 20 to 38 amino acids and comprising an amino acid sequence of 20 or more contiguous amino acids in the amino acid sequence of SEQ ID NO: 16.
[14] The peptide according to any one of items 11 to 13, consisting of contiguous.amino acids in the amino acid sequence of SEQ ID NO: 15.
[15] The peptide according to any one of items 11 to 14, comprising an amino acid sequence selected from SEQ ID NOs: 16 to 21.
[16] The peptide according to any one of items 11 to 15, consisting of an amino acid sequence selected from SEQ ID NOs: 16 to 21.
[17] The peptide according to item 1, which is
   a peptide consisting of 10 to 25 amino acids and comprising the amino acid sequence of SEQ ID NO: 9, or
   a peptide having an amino acid sequence that is different from the amino acid sequence of the former peptide in that one amino acid is substituted, deleted or added and being capable of activating a helper T-cell.
[18] The peptide according to item 17, consisting of 10 to 25 amino acids and comprising the amino acid sequence of SEQ ID NO: 9.
[19] The peptide according to item 17 or 18, consisting of contiguous amino acids in the amino acid sequence of SEQ ID NO: 2.
[20] The peptide according to any one of items 17 to 19, consisting of the amino acid sequence of SEQ ID NO: 9.
[21] A nucleic acid which encodes the peptide according to any one of items 1 to 20.
[22] An expression vector comprising the nucleic acid of item 21.
[23] An HLA multimer comprising the peptide according to any one of items 1 to 20 and an HLA class II molecule.
[24] An antigen-presenting cell presenting a complex of the peptide according to any one of items 1 to 20 and an HLA class II molecule.
[25] A helper T-cell capable of recognizing a complex of the peptide according to any one of items 1 to 20 and an HLA class II molecule.
[26] A pharmaceutical composition comprising the peptide according to any one of items 1 to 20, the nucleic acid according to item 21, the expression vector according to item 22, the HLA multimer according to item 23, the antigen-presenting cell according to item 24, or the helper T-cell according to item 25.
[27] The pharmaceutical composition according to item 26, further comprising a DNA methyltransferase inhibitor or used in combination with a DNA methyltransferase inhibitor.
[28] The pharmaceutical composition according to item 27, wherein the DNA methyltransferase inhibitor is selected from the group consisting of decitabine, guadecitabine, azacitidine, zebularine, tetrahydrouridine, tetrahydrouridine and derivatives thereof.
[29] The pharmaceutical composition according to item 27 or 29, wherein the DNA methyltransferase inhibitor is selected from the group consisting of decitabine and azacitidine.
[30] The pharmaceutical composition according to any one of items 26 to 29, for treating or preventing a cancer.
[31] The pharmaceutical composition according to any one of items 26 to 30, which is a cancer vaccine.
[32] The pharmaceutical composition according to item 30 or 31, wherein the cancer is hematological tumor, malignant melanoma, breast cancer, head and neck tumor, urinary tumor, esophageal cancer, liver cancer, lung cancer, or colon cancer.
[33] The pharmaceutical composition according to any one of items 30 to 32, wherein the cancer is lung cancer or colon cancer.
[34] A composition for activating a helper T-cell comprising the peptide according to any one of items 1 to 20, the nucleic acid according to item 21, the expression vector according to item 22, the HLA multimer according to item 23, or the antigen-presenting cell according to item 24.

The entire contents of the documents cited herein are incorporated herein by reference.

The following example does not restrict or limit the invention. The embodiments described above are non-limiting and may be modified without deviating from the scope of the invention as defined by the appended claims.

### EXAMPLES

### Test 1: Exploration of candidate genes

Genes re-upregulated upon the treatment with a DNA methyltransferase inhibitor (5-aza-2'-deoxycytidine (decitabine): 5-AZA) were identified by the following procedure. Cells of lung cancer cell line A549 were treated with 5-AZA (10 µM) for 3 days and the RNAs were extracted. Gene expression levels were measured using DNA microarrays. The criteria for identifying the candidate genes were that the expression level was close to 0 in the non-treated cells and increased by 30 times or more in the 5-AZA-treated cells. Some candidate genes were found, including SYCP3 (untreated: 3.0, 5-AZA treated: 340.9, fold-change: 112.3), SPESP1 (untreated: 1.7, 5-AZA treated: 65.3, fold-change: 38.8), and DAZL1 (untreated: 2.8, 5-AZA treated: 341.3, fold-change: 123.6).

### Test 2: Study of upregulated gene expressions of SYCP3, SPESP1, and DAZL1 induced by 5-AZA treatment

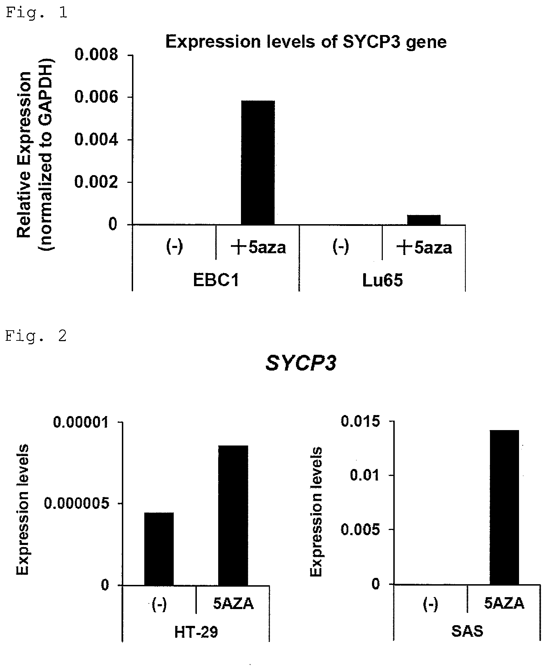

Cells of cancer cell lines (lung cancer cell line EBC1, lung cancer cell line Lu65, colon cancer cell line HT-29, and oral squamous cell carcinoma cell line SAS) were cultured in the complete medium (RPMI 1640 medium (nacalai tesque 30264-56) with 100 U/mL penicillin (Meiji Seika) and 100 µg/L streptomycin (Meiji Seika), added with 10% fetal bovine serum (biosera FB-1365/500) which had been immobilized at 56°C for 30 minutes) containing 5-AZA (10 µM) at the density of 2 x 10⁵ cells/well in 6-well plates (Falcon 353046) in an incubator (SANYO) set at 37°C, 5% CO₂, and 95% humidity for 3 days. The same incubator was used for all cell cultures in the following tests. The cells were washed with 2 mL of a phosphate-buffered saline (PBS, KANTO CHEMICAL CO., INC. 73111). The RNAs were extracted with RNeasy Mini Kit (Qiagen 74106), and the cDNAs were synthesized by using PrimeScript 1^{st} strand cDNA Synthesis Kit (Takara Bio 6110A), and the gene expression levels of GAPDH (Applied Biosystems Hs02786624_g1) and SYCP3 (Applied Biosystems Hs00538146_m1) were analyzed by real-time PCR using LightCycler480 (Roche). Each step was carried out in accordance with the description of the package inserts attached to each reagent. The results are shown in Figs. 1 and 2. SYCP3 was upregulated by the 5-AZA treatment in all the tested cell lines.

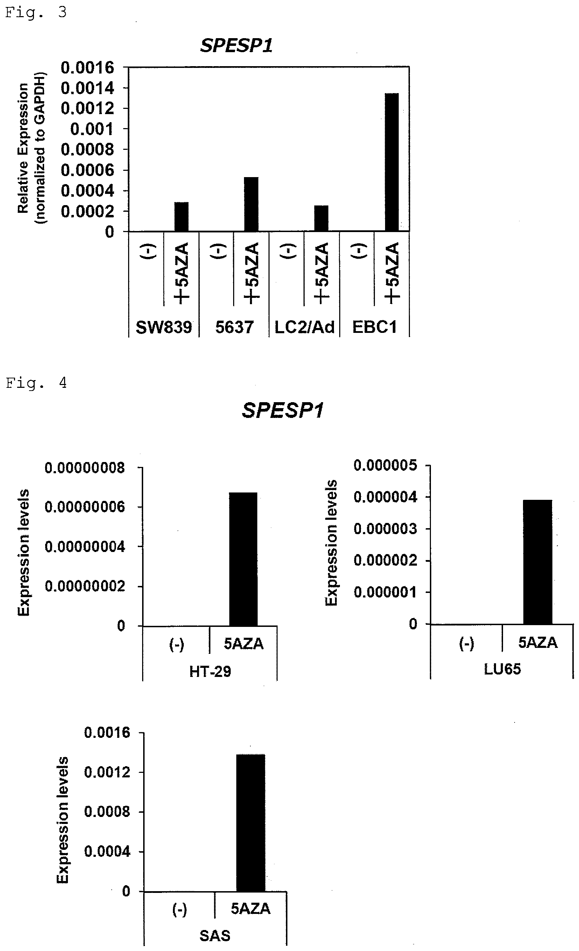

Similarly, cells of renal cancer cell line SW839, bladder cancer cell line 5637, lung adenocarcinoma cell line LC2/Ad, lung cancer cell line EBC1, colon cancer cell line HT-29, lung cancer cell line Lu65, and oral squamous cell carcinoma cell line SAS were cultured in the presence of 5-AZA, and the gene expression levels of GAPDH and SPESP1 (Applied Biosystems Hs00377364_m1) were analyzed. The results are shown in Figs. 3 and 4. SPESP1 was upregulated by the 5-AZA treatment in all the tested cell lines.

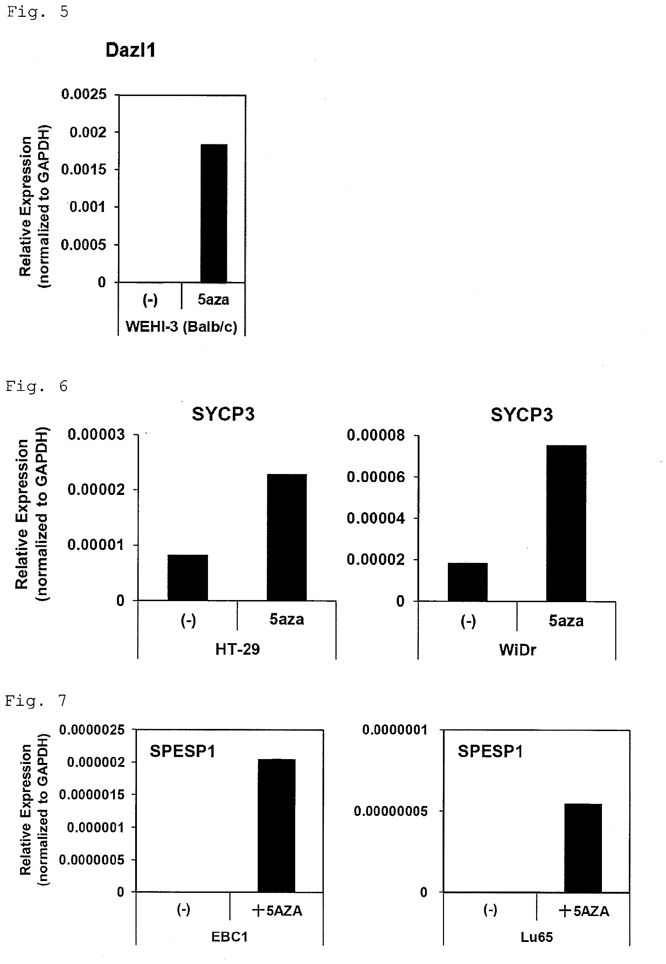

Similarly, cells of mouse leukemia cell line WEHI-3 (Balb/c) was cultured in the presence of 5-AZA, and the gene expression levels of mouse GAPDH (Applied Biosystems Mm99999915_g1) and mouse DAZL1 (Applied Biosystems Mm01273546_m1) were analyzed. The Results are shown in Fig. 5. DAZL1 was upregulated by the 5-AZA treatment in WEHI-3.

The amount of SYCP3 protein in EBC1 and Lu65 treated with 5-AZA was analyzed by Western blotting. An anti-SYCP3 antibody (Mouse anti-SCP3, BD Bioscience 611230) diluted at 1:200 was used as the primary antibody. Upregulation of SYCP3 by the 5-AZA treatment was also observed at the protein level.

Cells of mouse cancer cell lines (E0771:C57BL/6 mouse breast cancer cell line and C1498:C57BL/6 mouse acute myeloid leukemia cell line) were treated with 5-AZA (10 µM), and the amount of DAZL1 protein was analyzed by Western blotting. DAZL1 was upregulated by the 5-AZA treatment in both cell lines.

On the other hand, when cells of cancer cell lines EBC1, Lu65, and HT-29 were cultured in the presence of gemcitabine, an DNA synthesis inhibitor, the gene expression levels of SYCP3, SPESP1, and DAZL1 were equivalent to those of the controls. The results suggest that the upregulation of each gene observed in Test 2 is based on the unique effect of the DNA methyltransferase inhibitor.

### Test 3: Confirmation of upregulated gene expressions of SYCP3 and SPESP1 induced by 5-AZA treatment in immunodeficient mice

BALB/c nude mice (10 to 14 weeks old, CHARLES RIVER LABORATORIES JAPAN) were intradermally injected with cells of colorectal cancer cell line HT-29 (5 x 10⁵ cells) or WiDr (5 x 10⁵ cells) using Myjector (TERUMO SS_05M2913), and on Days 5, 10, 15, and 20, 200 µL of PBS containing 5-AZA (1.6 µg/g of mouse body weight) was intraperitoneally administered using Myjector. To the control mice, 200 µL of PBS was intraperitoneally administered. On Day 25 the mice were euthanized by intraperitoneally administering 200 µL of 500 µg/mL pentobarbital (nacalai tesque 02095-04), and tumor tissues were excised with scissors for dissection (NONAKARIKAKI Co., Ltd, 11301) and crushed by BioMasher II (Nippi, Incorporated, 320103). The gene expression levels of SYCP3 and GAPDH were analyzed by real-time PCR as described in Test 2. The results are shown in Fig. 6. SYCP3 was upregulated in the tumor tissues collected from the mice treated with 5-AZA, as compared with the mice treated with PBS alone.

Similarly, nude mice were intradermally injected with cells of lung cancer cell line EBC1 (5 × 10⁵ cells) or Lu65 (5 × 10⁵ cells), and 5-AZA or PBS (control) was intraperitoneally administered. The RNAs were extracted from the tumor tissues collected on Day 25, and the gene expression levels of SPESP1 and GAPDH were analyzed by real-time PCR. The results are shown in Fig. 7. SPESP1 was upregulated in the tumor tissues collected from the mice treated with 5-AZA, as compared with the mice treated with PBS alone.

### Test 4: Exploration of regions having HLA-binding amino acid sequences in the candidate proteins

To identify amino acid sequences in the proteins selected in Test 1 which is capable of binding to HLAs, each sequence was analyzed by a computer algorithm for the possibility of binding to five types of HLA frequently expressed in Japanese and Westerners, HLA-DRB1*01:01 (about 10%), HLA-DRB1*04:05 (about 25%), HLA-DRB1*09:01 (about 26%), HLA-DRB1*15:01 (about 15%), and HLA-DR53 (60% or more). Amino acid sequences containing SYCP3-A (SEQ ID NO: 8), SPESP1-B (SEQ ID NO: 9), and DAZL-1C (SEQ ID NO: 20) were identified.

### Test 5: Study of immunostimulatory ability of candidate amino acid sequences using HLA transgenic mice

HLA-A*02:01/DRB1*01:01 transgenic mice (A2.DR1-Tg mice, 10 to 16 weeks old; Pasteur Institute, France) were intradermally injected with SYCP3-A peptide (synthesized by GenScript) dissolved in 100 µL of PBS using Myjector on Days 0 and 10. The mice were euthanized by intraperitoneally administering 200 µL of 500 µg/mL pentobarbital on Day 15. After laparotomy, tumor-draining lymph nodes (dLNs) and spleens were collected and lymphocytes and splenocytes, respectively, were isolated.

The lymphocytes (3 x 10⁵ cells) and the splenocytes (5 x 10⁵ cells) were added to ELISPOT plates (EMD Millipore, MAHAS4510), and the cells were co-cultured in the presence of a control peptide (a peptide of 15 amino acid residues having a sequence not included in SYCP3) or SYCP3-A peptide selected in Test 4 (3 µg/mL) in the complete medium at 150 µL/well for 24 hours. In order to detect the cells producing IFN-γ specifically to SYCP3-A peptide, the ELISPOT assay was performed using a mouse IFN-γ ELISpot BASIC (ALP) kit (MABTECH 3321-2A). The procedure was in accordance with the description of the attached package insert. BCIP-NBT-plus substrate for ELISpot (MABTECH 3650-10) was used as a chromogenic substrate, and PBS-T was used for washing at each step. In addition, in order to confirm that the reaction is produced by CD4-positive T-cells, an anti-mouse CD4 antibody (BioLegend 100435) or an anti-mouse CD8 antibody (BioLegend 100735) was added to some cultures at the final concentration of 5 µg/mL.

The results are shown in the table below. A specific T-cell response was induced by the stimulation of SYCP3-A peptide. The fact that the reaction was inhibited by the antibody against CD4 indicates that the response was induced by CD4-positive T-cells.

**[Table 1]**

| SYCP3-A peptide-specific IFN-γ production | | | | |
|---|---|---|---|---|
| | 1 | 2 | 3 | 4 |
| SYCP3-A | - | + | + | + |
| anti-CD8 antibody | - | - | + | - |
| anti-CD4 antibody | - | - | - | + |
| IFN-γ producing cells | - | + | + | - |

Furthermore, in order to identify the region in SYCP3-A peptide with which CD4-positive T-cells interact, partial peptides having contiguous 12 amino acids in the sequence of SYCP3-A peptide wherein the starting amino acids are sequentially shifted by one amino acid were synthesized by using Sigma-aldrich Pepscreen (table below).

**[Table 2]**

| SYCP3-A | QQQKILQQSRIVQSQRLKT (SEQ ID NO: 8) |
|---|---|
| T1 | QQQKILQQSRIV (SEQ ID NO: 10) |
| T2 | QQKILQQSRIVQ (SEQ ID NO: 7) |
| T3 | QKILQQSRIVQS (SEQ ID NO: 6) |
| T4 | KILQQSRIVQSQ (SEQ ID NO: 5) |
| T5 | ILQQSRIVQSQR (SEQ ID NO: 11) |
| T6 | LQQSRIVQSQRL (SEQ ID NO: 12) |
| T7 | QQSRIVQSQRLK (SEQ ID NO: 13) |
| T8 | QSRIVQSQRLKT (SEQ ID NO: 14) |

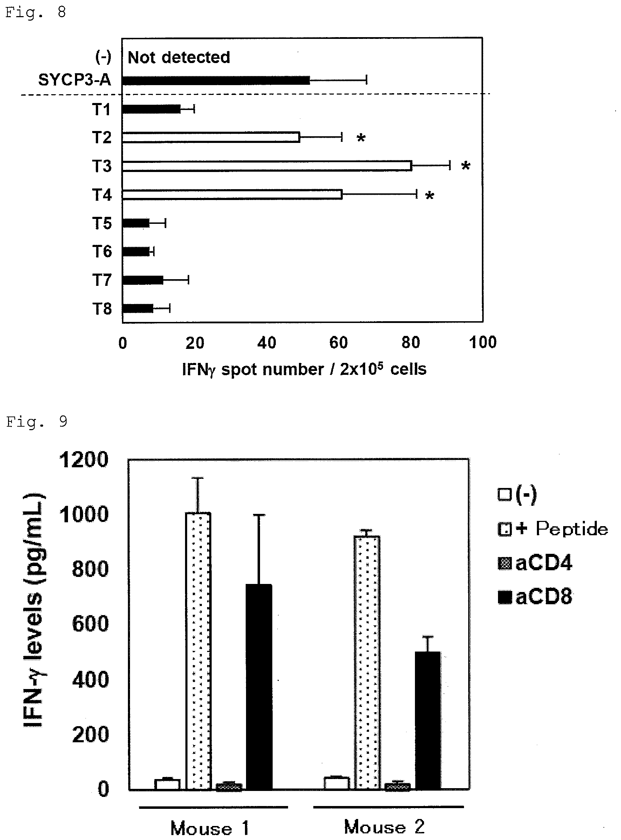

As described above, A2.DR1-Tg mice were vaccinated with these partial peptides, dLNs and spleens were collected from each mouse, lymphocytes and splenocytes were isolated, the cells were stimulated with each partial peptide (3 µg/mL) for 24 hours, and the T-cell responses were determined by ELISPOT. The results are shown in Fig. 8. CD4-positive T-cells responded to the partial peptides T2, T3, and T4. The results suggest that the region in SYCP3-A peptide with which CD4-positive T-cells interact is the amino acid sequence KILQQSRIVQ common in the T2, T3, and T4 peptides.

### Test 6: Study of immunostimulatory ability of DAZL1 candidate peptides using mice

Two BALB/cAnNCrlCrlj mice (Balb/c, 10 to 16 weeks old, Charles river) were intradermally injected with 100 µg/50 µL DAZL-1C peptide (DVQKIVESQINFHGKKLKLGPAIRKQNLC (SEQ ID NO: 20): synthesized by GenScript) dissolved in phosphate-buffered saline (PBS, KANTO CHEMICAL CO., INC. 73111) using Myjector (TERUMO SS_05M2913) on Days 0 and 10. The mice were euthanized by intraperitoneally administering 200 µL of 500 µg/mL pentobarbital (nacalai tesque 02095-04) on Day 12. After laparotomy, dLNs and spleens were collected and lymphocytes and splenocytes, respectively, were isolated.

The lymphocytes (3 x 10⁵ cells) and the splenocytes (5 x 10⁵ cells) were added to 96-well flat bottom culture plates (Falcon 353072), and the cells were co-cultured in the presence of a control peptide or DAZL-1C peptide (2.5 µg/mL) in the complete medium at 200 pL/well for 24 hours. In order to detect IFN-γ production specific to DAZL-1C peptide, each 100 µL of the culture supernatants after 24 hours were collected, and the IFN-γ concentrations were determined using an ELISA set (BD Biosciences 551866) in accordance with the description of the attached package insert. In order to confirm that the reaction is produced by CD4-positive T-cells, an anti-mouse CD4 antibody (aCD4; BioLegend 100435) or an anti-mouse CD8 antibody (aCD8; BioLegend 100735) was added to some cultures at the final concentration of 5 µg/mL.

The results are shown in Fig. 9. The fact that the IFN-γ production specific to DAZL-1C peptide was inhibited by the anti-CD4 antibody indicates that DAZL-1C peptide activates helper T-cells in mice.

### Test 7: Induction of SYCP3-A or SPESP1-B peptide-specific helper T (Th) cells using peripheral blood mononuclear cells (PBMCs) from healthy donors

PBMCs were collected from peripheral blood samples of healthy donors by a density gradient separation method using Lymphoprep (Alere Technologies AS 1114547). CD14 positive cells were separated from PBMCs using a magnetic cell separation system (Miltenyi 130-050-201). Differentiation into dendritic cells (DCs) was induced by culturing the cells in the presence of 50 ng/mL GM-CSF (peprotech AF-300-03) and 50 ng/mL IL-4 (peprotech AF-200-04) with 3 mL of a culture medium for human cells in 6-well culture plates (Falcon 353046) for 7 days. The culture medium was AIM-V medium (ThermoFisher SCIENTIFIC 0870112DK) supplemented with 3% of human AB serum (Innovative RESEARCH IPLA-SERAB) inactivated at 56°C for 30 minutes. Similarly, CD4-positive T-cells were isolated from PBMCs (Miltenyi 130-045-101), and 1 × 10⁵ CD4-positive T-cells were co-cultured with 5 × 10⁴ DCs in the presence of SYCP3-A or SPESP1-B peptide (3 µg/mL) in 96-well flat bottom culture plates (Falcon 353072) at the volume of 200 µL. After 7 days, in order to stimulate the CD4-positive T-cells with the peptide, 100 µL of the culture supernatant was .removed from each well, and then SYCP3-A or SPESP1-B peptide (3 µg/mL) and PBMCs (2 × 10⁵) inactivated by gamma-irradiation (40 Gy) were added at the volume of 100 µL. After 2 days, 50 µL of the culture supernatant was removed, and 50 µL of IL-2 (Imunace35, Shionogi) was added at the final concentration of 10 U/mL. For continuous proliferation of the activated CD4-positive T-cells, the cells (1 x 10⁶ cells) were stimulated with SYCP3-A or SPESP1-B peptide and the inactivated PBMCs (1 x 10⁶ cells) every other week and used for the experiments described below.

### Test 8: HLA restriction of SYCP3-A peptide-specific helper T-cell lines

To study the specific reactivity of the proliferated CD4-positive T-cells toward SYCP3-A peptide, the CD4-positive T-cells (5 × 10⁴ cells) and PBMCs (1 × 10⁵ cells) were co-cultured in the presence of SYCP3-A peptide (3 µg/mL) in 96-well flat bottom culture plates using 200 µL of the culture medium for human cells. In order to study the HLA-restriction of the CD4-positive T-cells, an anti-DR antibody (BioLegend 307612) or an anti-HLA-class I antibody (BioLegend 311412) as a control was added to some cultures at the final concentration of 5 µg/mL. After 24 to 48 hours 100 µL of the culture supernatants were collected from each well, and the IFN-γ concentrations were determined using an ELISA kit (BD Biosciences 555142) in accordance with the description of the attached package insert.

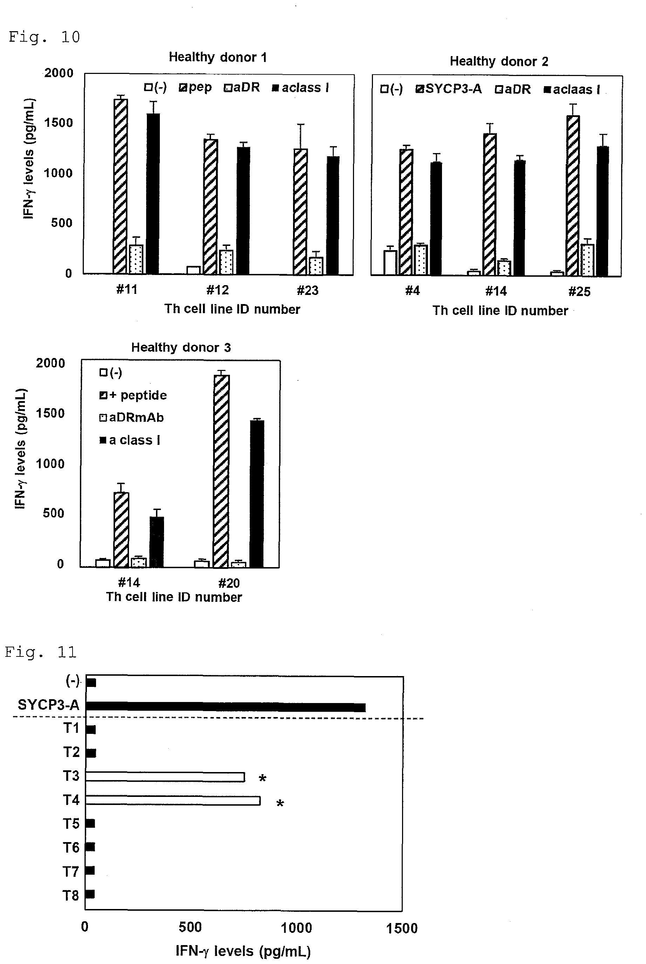

The results are shown in Fig. 10. A plurality of SYCP3-A-specific Th cell clones were established from the three healthy donors. The peptide-specific IFN-γ production was suppressed when the anti-HLA-DR antibody (aDR) was added to the clones. The results indicate that SYCP3-A peptide stimulates Th cells with HLA-DR-restriction.

Furthermore, in order to identify the HLA type to which the peptide is restricted, the CD4-positive T-cells (5 × 10⁴ cells) and 3 × 10⁴ cells of a mouse fibroblast cell line having HLA-DR4, DR8, DR9, or DR53 gene (L-DR4, L-DR8, L-DR9, or L-DR53) were co-cultured in the presence of SYCP3-A peptide (3 µg/mL) in 96-well flat bottom culture plates using 200 µL of the culture medium for human cells, and after 24 to 48 hours the IFN-γ concentrations in the culture supernatants were determined as described above.

All tested SYCP3-A peptide-specific Th cells showed strong peptide-specific reactions (high expression levels of INF-γ) to L-DR53 cells. SYCP3-A peptide also showed some reactivity even in the samples having no DR53 allele. The results suggest that the peptide is effective for some alleles other than DR53.

In order to identify the minimum sequence in SYCP3-A peptide required for the Th cell recognition, 5 × 10⁴ SYCP3-A peptide-specific Th cells (cell line ID number #14 from healthy donor 3) and 1 ×10⁵ PBMCs derived from the same donor were co-cultured in the presence of any one of the partial SYCP3-A peptides T1 to T8 (see Test 5) at the concentration of 3 µg/mL in 96-well flat bottom culture plates using 200 µL of the culture medium for human cells. The results are shown in Fig. 11. Increased IFN-γ production was observed for T3 and T4 peptides. The results suggest that the minimum sequence required for recognition by the SYCP3-A peptide-specific Th cells is the amino acid sequence KILQQSRIVQS common in T3 and T4.

The peptides listed in the table below were similarly tested.

**[Table 3]**

| SYCP3-A | QQQKILQQSRIVQSQRLKT (SEQ ID NO: 8) |
|---|---|
| SYCP3-B-1) | RQQQKILQQSRIVQSQRLKT (SEQ ID NO: 22) |
| SYCP3-B-2) | LNMFRQQQKILQQSRIVQSQRLKT (SEQ ID NO: 23) |
| SYCP3-B-3) | QQQKILQQSRIVQSQRLKTI (SEQ ID NO: 24) |
| SYCP3-B-4) | QQQKILQQSRIVQSQRLKTIKQLY (SEQ ID NO: 25) |
| SYCP3-B-5) | Ac-QQQKILQQSRIVQSQRLKT (Ac: acetyl group) |

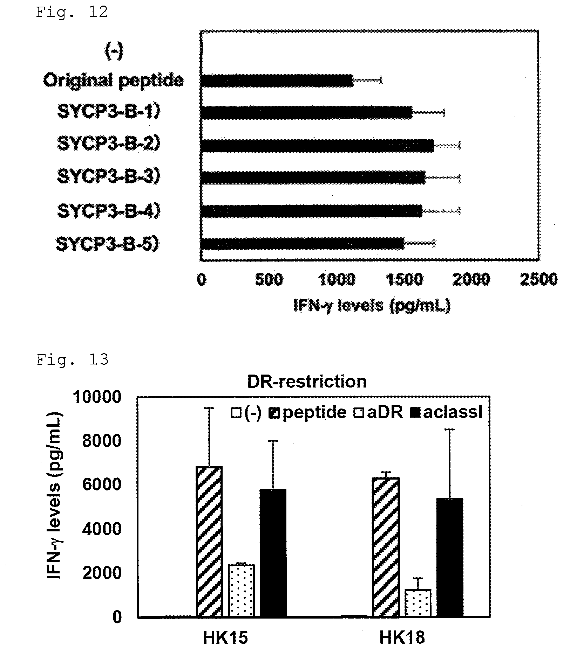

The results are shown in Fig. 12. Increased IFN-γ production was observed for all peptides. The results suggest that the reactivity is retained even if one or more amino acids are added to N- or C-terminus of the minimum sequence. Addition of an acetyl group did not alter the reactivity. This suggests modification of the peptide is allowed. No difference in the reactivity was found between the two clones.

The 11th isoleucine residue of SYCP3-A peptide was substituted with a valine residue and the peptide (SEQ ID NO: 31) was similarly tested. The reactivity was not decreased. This suggests a certain amino acid mutation is allowed in the region of SYCP3-A peptide that interacts with CD4-positive T-cells.

### Test 9: HLA restriction of SPESP1-B peptide-specific helper T-cell lines

The IFN-γ concentrations of the culture supernatants were determined in the same manner as in Test 8, except that SPESP1-B peptide was used instead of SYCP3-A peptide. The results are shown in Fig. 13. Two SPESP1-B-specific Th cell clones (HK15 and HK18) were established from one healthy donor. The peptide-specific IFN-γ production was suppressed when the anti-HLA-DR antibody (aDR) was added to the clones. The results indicate that the SPESP1-B peptide stimulates Th cells with HLA-DR-restriction. Further experiments using L-DR53 as described in Test 8 revealed that SPESP1-B peptide binds to HLA-DR53 and stimulates Th cells.

In order to study the reactivity of the SPESP1-B peptide-specific Th cells toward the peptide, the Th cells were stimulated with SPESP1-B peptide serially diluted to 0.0003 to 30 µg/mL. SPESP1-B peptide induced sufficient IFN-γ production even at a low concentration (0.0003 µg/mL).

### Test 10: Induction of DAZL-1 peptide-specific Th cells and study of the HLA restriction

DAZL-1 peptide-specific Th cells were induced in the same manner as in Test 7, except that partial DAZL-1 peptide p11, p12, or p13 (Table 4) was used instead of SYCP3-A or SPESP1-B peptide.

**[Table 4]**

| Peptide sequences | |
|---|---|
| **p11** | **DVQKIVESQINFHGKKLKLG** (SEQ ID NO: 17) |
| **p12** | **INFHGKKLKLGPAIRKQNLC** (SEQ ID NO: 18) |
| **p13** | **LGPAIRKQNLCAYHVQPRPL** (SEQ ID NO: 19) |

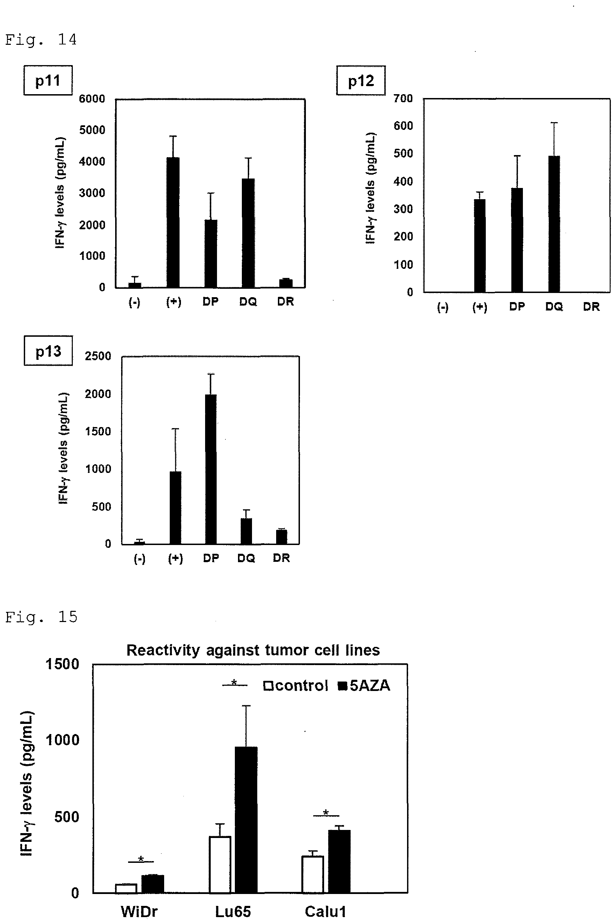

The IFN-γ concentrations of the culture supernatants were determined in the same manner as in Test 8, except that partial DAZL-1 peptide p11, p12, or p13 was used instead of SYCP3-A peptide. In order to study the HLA-restriction, an anti-HLA-DP antibody, an anti-HLA-DQ antibody (SPV-L3: Abcam ab85614), and an anti-HLA-DR antibody (BRAFB6: Santa Cluz sc-33719) were used. The results are shown in Fig. 14. The peptide-specific IFN-γ production was suppressed when the anti-HLA-DR antibody (aDR) was used. The results indicate that the partial DAZL-1 peptides stimulate Th cells with HLA-DR-restriction. The IFN-γ production induced with p13 was also suppressed with the anti-HLA-DQ antibody. The results suggest that peptides derived from DAZL-1 are effective to diverse HLAs.

Further experiments using L-DR4, L-DR8, L-DR9, L-DR15, and L-DR53 as in Test 8 revealed that p11, p12, and p13 bind to HLA-DR4/9/53, HLA-DR15, and HLA-DR8, respectively, to stimulate Th cells.

### Test 11: Reactivity of SYCP3-A peptide-specific CD4-positive T-cells toward cancer cells

In order to study the reactivity of SYCP3-A peptide-specific CD4-positive T-cells toward cells of cancer cell lines, DR53-positive cancer cell lines (WiDr, Lu65, and Calu1) were used. The cancer cells were cultured in 6-well culture plates for 3 days using 2 mL of the complete medium containing 10 µM 5-AZA and 500 U/mL IFN-γ (Immunomax-γ for injection 50, Shionogi), which can induce expression of HLA class II molecules on the surfaces of cancer cells. The culture plates were thoroughly washed with PBS, 1 mL of 5 mM EDTA (ethylenediaminetetraacetic acid, nacalai tesque 14347-21) was added to suspend the cells, and the cells were recovered. In the same manner as in Test 8, 1 × 10⁴ cells of each cell line and 5 × 10⁴ CD4-positive T-cells were co-cultured in 96-well flat bottom culture plates using 200 µL of the culture medium for human cells. In order to confirm that the reactivity depends on HLA-DR, the anti-HLA-DR antibody was added to the cultures at the final concentration of 5 µg/mL. After 24 hours 100 µL of the culture supernatants were collected from each well, and the IFN-γ concentrations were determined using the ELISA kit.

The results are shown in Fig. 15. The 5-AZA treatment increased the IFN-γ production. The results indicate Th cells activated with SYCP3-A peptide effectively react toward DR53-positive cancer cells treated with 5-AZA.

### Test 12: Tumor growth inhibitory effect of DNA methyltransferase inhibitor and SYCP3-specific Th cells in immunodeficient mice

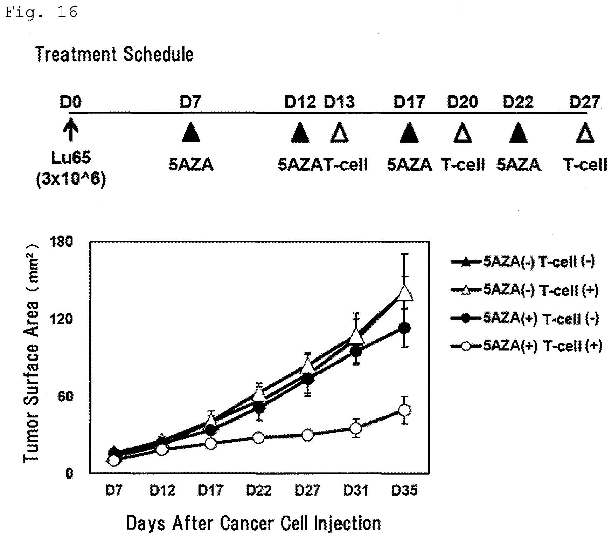

BALB/c nude mice (10 to 14 weeks old, CHARLES RIVER LABORATORIES JAPAN) were intradermally injected with 3 x 10⁶ cells of lung cancer cell line Lu65 using Myjector, and on Days 7, 12, 17, and 22, 200 µL of PBS containing 5-AZA (150 nmol/g of mouse body weight) was intraperitoneally administered using Myjector. To the control mice, 200 µL of PBS was intraperitoneally administered. On Days 13, 20, and 27, 200 µL of SYCP3-specific human Th cells (3 to 5 x 10⁶ cells) were administered by tail vein administration. To the control mice, 200 µL of PBS was administered by tail vein administration. The tumor surface areas were measured over time. The results are shown in Fig. 16. No effect was observed for 5-AZA alone, whereas the tumor growth suppressing effect was observed for the combination of 5-AZA and the SYCP3-specific human Th cells.

### INDUSTRIAL APPLICABILITY

The cancer antigen peptide disclosed herein activates helper T-cells specific for the peptide, thus can be used as a cancer vaccine. The peptide binds to HLAs including HLA-DR53, which is shared with high frequency, and thus will be effective in many cancer patients.

## Claims

1. A peptide consisting of 10 to 45 amino acids and comprising
the amino acid sequence of KILQQSRIVQX, wherein X is absent or S;
an amino acid sequence of 10 or more contiguous amino acids in the amino acid sequence of DVQKIVESQINFHGKKLKLGPAIRKQNLCAYHVQPRPL (SEQ ID NO: 16); or the amino acid sequence of QNLNHYIQVLENLVRSVPS (SEQ ID NO: 9), or
a peptide having an amino acid sequence that is different from the amino acid sequence of the former peptide in that 1 to 3 amino acids are substituted, deleted or added and being capable of activating a helper T-cell.

2. The peptide according to claim 1, which is
a peptide consisting of 10 to 25 amino acids and comprising the amino acid sequence of KILQQSRIVQX, wherein X is absent or S,
or
a peptide having an amino acid sequence that is different from the amino acid sequence of the former peptide in that one amino acid is substituted, deleted or added and being capable of activating a helper T-cell.

3. The peptide according to claim 2, consisting of 10 to 25 amino acids and comprising the amino acid sequence of KILQQSRIVQX, wherein X is absent or S.

4. The peptide according to claim 3, consisting of contiguous amino acids in the amino acid sequence of SEQ ID NO: 1.

5. The peptide according to claim 3 or 4, comprising an amino acid sequence selected from the group consisting of KILQQSRIVQSQ (SEQ ID NO: 5), QKILQQSRIVQS (SEQ ID NO: 6), and QQKILQQSRIVQ (SEQ ID NO: 7).

6. The peptide according to claim 1, consisting of 10 to 45 amino acids and comprising an amino acid sequence of 10 or more contiguous amino acids in the amino acid sequence of DVQKIVESQINFHGKKLKLGPAIRKQNLCAYHVQPRPL (SEQ ID NO: 16).

7. The peptide according to claim 1 or 6, comprising an amino acid sequence selected from the group consisting of DVQKIVESQINFHGKKLKLG (SEQ ID NO: 17), INFHGKKLKLGPAIRKQNLC (SEQ ID NO: 18), and LGPAIRKQNLCAYHVQPRPL (SEQ ID NO: 19).

8. The peptide according to any one of claims 1, 6, and 7, consisting of an amino acid sequence selected from the group consisting of DVQKIVESQINFHGKKLKLG (SEQ ID NO: 17), INFHGKKLKLGPAIRKQNLC (SEQ ID NO: 18), and LGPAIRKQNLCAYHVQPRPL (SEQ ID NO: 19).

9. The peptide according to claim 1, consisting of 19 to 25 amino acids and comprising the amino acid sequence of QNLNHYIQVLENLVRSVPS (SEQ ID NO: 9).

10. The peptide according to claim 1 or 9, consisting of the amino acid sequence of QNLNHYIQVLENLVRSVPS (SEQ ID NO: 9).

11. A nucleic acid which encodes the peptide according to any one of claims 1 to 10.

12. A pharmaceutical composition comprising the peptide according to any one of claims 1 to 10, the nucleic acid according to claim 11, an expression vector comprising the nucleic acid according to claim 11, an HLA multimer comprising the peptide according to any one of claims 1 to 10 and an HLA class II molecule, an antigen-presenting cell presenting a complex of the peptide according to any one of claims 1 to 10 and an HLA class II molecule, or a helper T-cell capable of recognizing a complex of the peptide according to any one of claims 1 to 10 and an HLA class II molecule.

13. The pharmaceutical composition according to claim 12, further comprising a DNA methyltransferase inhibitor or used in combination with a DNA methyltransferase inhibitor.

14. The pharmaceutical composition according to claim 12 or 13, for treating or preventing a cancer.

15. The pharmaceutical composition according to claim 12 or 13, which is a cancer vaccine.


SEQUENCE LISTING

<110> OTSUKA PHARMACEUTICAL CO., LTD.
NATIONAL UNIVERSITY CORPORATION ASAHIKAWA MEDICAL
UNIVERSITY

<120> TUMOR ANTIGEN PEPTIDE

<130> 674674

<150> JP 2018-024972
<151> 2018-02-15

<160> 35

<170> PatentIn version 3.5

<210> 1
<211> 236
<212> PRT
<213> Homo sapiens

<400> 1

Met Val Ser Ser Gly Lys Lys Tyr Ser Arg Lys Ser Gly Lys Pro Ser
1               5                   10                  15

Val Glu Asp Gln Phe Thr Arg Ala Tyr Asp Phe Glu Thr Glu Asp Lys
            20                  25                  30

Lys Asp Leu Ser Gly Ser Glu Glu Asp Val Ile Glu Gly Lys Thr Ala
        35                  40                  45

Val Ile Glu Lys Arg Arg Lys Lys Arg Ser Ser Ala Gly Val Val Glu
    50                  55                  60

Asp Met Gly Gly Glu Val Gln Asn Met Leu Glu Gly Val Gly Val Asp
65                  70                  75                  80

Ile Asn Lys Ala Leu Leu Ala Lys Arg Lys Arg Leu Glu Met Tyr Thr
                85                  90                  95

Lys Ala Ser Leu Lys Thr Ser Asn Gln Lys Ile Glu His Val Trp Lys
            100                 105                 110

Thr Gln Gln Asp Gln Arg Gln Lys Leu Asn Gln Glu Tyr Ser Gln Gln
        115                 120                 125

Phe Leu Thr Leu Phe Gln Gln Trp Asp Leu Asp Met Gln Lys Ala Glu
    130                 135                 140

Glu Gln Glu Glu Lys Ile Leu Asn Met Phe Arg Gln Gln Gln Lys Ile
145                 150                 155                 160

Leu Gln Gln Ser Arg Ile Val Gln Ser Gln Arg Leu Lys Thr Ile Lys
                165                 170                 175

Gln Leu Tyr Glu Gln Phe Ile Lys Ser Met Glu Glu Leu Glu Lys Asn
            180                 185                 190

His Asp Asn Leu Leu Thr Gly Ala Gln Asn Glu Phe Lys Lys Glu Met
        195                 200                 205

Ala Met Leu Gln Lys Lys Ile Met Met Glu Thr Gln Gln Gln Glu Ile
    210                 215                 220

Ala Ser Val Arg Lys Ser Leu Gln Ser Met Leu Phe
225                 230                 235

<210>  2
<211>  331
<212>  PRT
<213>  Homo sapiens

<400>  2

Tyr Pro Ser Ile Thr Val Thr Pro Asp Glu Glu Gln Asn Leu Asn His
1               5                   10                  15

Tyr Ile Gln Val Leu Glu Asn Leu Val Arg Ser Val Pro Ser Gly Glu
            20                  25                  30

Pro Gly Arg Glu Lys Lys Ser Asn Ser Pro Lys His Val Tyr Ser Ile
        35                  40                  45

Ala Ser Lys Gly Ser Lys Phe Lys Glu Leu Val Thr His Gly Asp Ala
    50                  55                  60

Ser Thr Glu Asn Asp Val Leu Thr Asn Pro Ile Ser Glu Glu Thr Thr
65                  70                  75                  80

Thr Phe Pro Thr Gly Gly Phe Thr Pro Glu Ile Gly Lys Lys Lys His
                85                  90                  95

Thr Glu Ser Thr Pro Phe Trp Ser Ile Lys Pro Asn Asn Val Ser Ile
            100                 105                 110

Val Leu His Ala Glu Glu Pro Tyr Ile Glu Asn Glu Glu Pro Glu Pro
        115                 120                 125

Glu Pro Glu Pro Ala Ala Lys Gln Thr Glu Ala Pro Arg Met Leu Pro
    130                 135                 140

Val Val Thr Glu Ser Ser Thr Ser Pro Tyr Val Thr Ser Tyr Lys Ser
145                 150                 155                 160

Pro Val Thr Thr Leu Asp Lys Ser Thr Gly Ile Gly Ile Ser Thr Glu
                165                 170                 175

Ser Glu Asp Val Pro Gln Leu Ser Gly Glu Thr Ala Ile Glu Lys Pro
            180                 185                 190

Glu Glu Phe Gly Lys His Pro Glu Ser Trp Asn Asn Asp Asp Ile Leu
        195                 200                 205

Lys Lys Ile Leu Asp Ile Asn Ser Gln Val Gln Gln Ala Leu Leu Ser
    210                 215                 220

Asp Thr Ser Asn Pro Ala Tyr Arg Glu Asp Ile Glu Ala Ser Lys Asp
225                 230                 235                 240

His Leu Lys Arg Ser Leu Ala Leu Ala Ala Ala Ala Glu His Lys Leu
                245                 250                 255

Lys Thr Met Tyr Lys Ser Gln Leu Leu Pro Val Gly Arg Thr Ser Asn
            260                 265                 270

Lys Ile Asp Asp Ile Glu Thr Val Ile Asn Met Leu Cys Asn Ser Arg
        275                 280                 285

Ser Lys Leu Tyr Glu Tyr Leu Asp Ile Lys Cys Val Pro Pro Glu Met
    290                 295                 300

Arg Glu Lys Ala Ala Thr Val Phe Asn Thr Leu Lys Asn Met Cys Arg
305                 310                 315                 320

Ser Arg Arg Val Thr Ala Leu Leu Lys Val Tyr
                325                 330


<210>  3
<211>  10
<212>  PRT
<213>  Homo sapiens

<400>  3

Lys Ile Leu Gln Gln Ser Arg Ile Val Gln
1               5                   10


<210>  4
<211>  11
<212>  PRT

<213>  Homo sapiens

<400>  4

Lys Ile Leu Gln Gln Ser Arg Ile Val Gln Ser
1               5                   10


<210>  5
<211>  12
<212>  PRT
<213>  Homo sapiens

<400>  5

Lys Ile Leu Gln Gln Ser Arg Ile Val Gln Ser Gln
1               5                   10


<210>  6
<211>  12
<212>  PRT
<213>  Homo sapiens

<400>  6

Gln Lys Ile Leu Gln Gln Ser Arg Ile Val Gln Ser
1               5                   10


<210>  7
<211>  12
<212>  PRT
<213>  Homo sapiens

<400>  7

Gln Gln Lys Ile Leu Gln Gln Ser Arg Ile Val Gln
1               5                   10


<210>  8
<211>  19
<212>  PRT
<213>  Homo sapiens

<400>  8

Gln Gln Gln Lys Ile Leu Gln Gln Ser Arg Ile Val Gln Ser Gln Arg
1               5                   10                  15


Leu Lys Thr


<210>  9
<211>  19
<212>  PRT
<213>  Homo sapiens

<400>  9

Gln Asn Leu Asn His Tyr Ile Gln Val Leu Glu Asn Leu Val Arg Ser
1               5                   10                  15

Val Pro Ser

<210>  10
<211>  12
<212>  PRT
<213>  Homo sapiens

<400>  10

Gln Gln Gln Lys Ile Leu Gln Gln Ser Arg Ile Val
1               5                   10

<210>  11
<211>  12
<212>  PRT
<213>  Homo sapiens

<400>  11

Ile Leu Gln Gln Ser Arg Ile Val Gln Ser Gln Arg
1               5                   10

<210>  12
<211>  12
<212>  PRT
<213>  Homo sapiens

<400>  12

Leu Gln Gln Ser Arg Ile Val Gln Ser Gln Arg Leu
1               5                   10

<210>  13
<211>  12
<212>  PRT
<213>  Homo sapiens

<400>  13

Gln Gln Ser Arg Ile Val Gln Ser Gln Arg Leu Lys
1               5                   10

<210>  14
<211>  12
<212>  PRT
<213>  Homo sapiens

<400>  14

Gln Ser Arg Ile Val Gln Ser Gln Arg Leu Lys Thr
1               5                   10

<210>  15
<211>  295
<212>  PRT
<213>  Homo sapiens

<400>  15

Met Ser Thr Ala Asn Pro Glu Thr Pro Asn Ser Thr Ile Ser Arg Glu
1               5                   10                  15

Ala Ser Thr Gln Ser Ser Ser Ala Ala Thr Ser Gln Gly Tyr Ile Leu
            20                  25                  30

Pro Glu Gly Lys Ile Met Pro Asn Thr Val Phe Val Gly Gly Ile Asp
        35                  40                  45

Val Arg Met Asp Glu Thr Glu Ile Arg Ser Phe Phe Ala Arg Tyr Gly
    50                  55                  60

Ser Val Lys Glu Val Lys Ile Ile Thr Asp Arg Thr Gly Val Ser Lys
65                  70                  75                  80

Gly Tyr Gly Phe Val Ser Phe Phe Asn Asp Val Asp Val Gln Lys Ile
                85                  90                  95

Val Glu Ser Gln Ile Asn Phe His Gly Lys Lys Leu Lys Leu Gly Pro
            100                 105                 110

Ala Ile Arg Lys Gln Asn Leu Cys Ala Tyr His Val Gln Pro Arg Pro
        115                 120                 125

Leu Val Phe Asn His Pro Pro Pro Pro Gln Phe Gln Asn Val Trp Thr
    130                 135                 140

Asn Pro Asn Thr Glu Thr Tyr Met Gln Pro Thr Thr Thr Met Asn Pro
145                 150                 155                 160

Ile Thr Gln Tyr Val Gln Ala Tyr Pro Thr Tyr Pro Asn Ser Pro Val
                165                 170                 175

Gln Val Ile Thr Gly Tyr Gln Leu Pro Val Tyr Asn Tyr Gln Met Pro
            180                 185                 190

Pro Gln Trp Pro Val Gly Glu Gln Arg Ser Tyr Val Val Pro Pro Ala
        195                 200                 205

Tyr Ser Ala Val Asn Tyr His Cys Asn Glu Val Asp Pro Gly Ala Glu
    210                 215                 220

Val Val Pro Asn Glu Cys Ser Val His Glu Ala Thr Pro Pro Ser Gly
225                 230                 235                 240

Asn Gly Pro Gln Lys Lys Ser Val Asp Arg Ser Ile Gln Thr Val Val
                245                 250                 255

Ser Cys Leu Phe Asn Pro Glu Asn Arg Leu Arg Asn Ser Val Val Thr
            260                 265                 270

Gln Asp Asp Tyr Phe Lys Asp Lys Arg Val His His Phe Arg Arg Ser
        275                 280                 285

Arg Ala Met Leu Lys Ser Val
    290                 295

<210>  16
<211>  38
<212>  PRT
<213>  Homo sapiens

<400>  16

Asp Val Gln Lys Ile Val Glu Ser Gln Ile Asn Phe His Gly Lys Lys
1               5                   10                  15

Leu Lys Leu Gly Pro Ala Ile Arg Lys Gln Asn Leu Cys Ala Tyr His
            20                  25                  30

Val Gln Pro Arg Pro Leu
        35

<210>  17
<211>  20
<212>  PRT
<213>  Homo sapiens

<400>  17

Asp Val Gln Lys Ile Val Glu Ser Gln Ile Asn Phe His Gly Lys Lys
1               5                   10                  15

Leu Lys Leu Gly
            20

<210>  18
<211>  20
<212>  PRT
<213>  Homo sapiens

<400>  18

Ile Asn Phe His Gly Lys Lys Leu Lys Leu Gly Pro Ala Ile Arg Lys
1               5                   10                  15

Gln Asn Leu Cys
            20

<210>  19
<211>  20
<212>  PRT
<213>  Homo sapiens

<400>  19

Leu Gly Pro Ala Ile Arg Lys Gln Asn Leu Cys Ala Tyr His Val Gln
1               5                   10                  15

Pro Arg Pro Leu
            20

<210>  20
<211>  29
<212>  PRT
<213>  Homo sapiens

<400>  20

Asp Val Gln Lys Ile Val Glu Ser Gln Ile Asn Phe His Gly Lys Lys
1               5                   10                  15

Leu Lys Leu Gly Pro Ala Ile Arg Lys Gln Asn Leu Cys
            20                  25

<210>  21
<211>  29
<212>  PRT
<213>  Homo sapiens

<400>  21

Ile Asn Phe His Gly Lys Lys Leu Lys Leu Gly Pro Ala Ile Arg Lys
1               5                   10                  15

Gln Asn Leu Cys Ala Tyr His Val Gln Pro Arg Pro Leu
            20                  25

<210>  22
<211>  20
<212>  PRT
<213>  Homo sapiens

<400>  22

Arg Gln Gln Gln Lys Ile Leu Gln Gln Ser Arg Ile Val Gln Ser Gln
1               5                   10                  15

Arg Leu Lys Thr

            20

<210>  23
<211>  24
<212>  PRT
<213>  Homo sapiens

<400>  23

Leu Asn Met Phe Arg Gln Gln Gln Lys Ile Leu Gln Gln Ser Arg Ile
1               5                   10                  15

Val Gln Ser Gln Arg Leu Lys Thr
            20

<210>  24
<211>  20
<212>  PRT
<213>  Homo sapiens

<400>  24

Gln Gln Gln Lys Ile Leu Gln Gln Ser Arg Ile Val Gln Ser Gln Arg
1               5                   10                  15

Leu Lys Thr Ile
            20

<210>  25
<211>  24
<212>  PRT
<213>  Homo sapiens

<400>  25

Gln Gln Gln Lys Ile Leu Gln Gln Ser Arg Ile Val Gln Ser Gln Arg
1               5                   10                  15

Leu Lys Thr Ile Lys Gln Leu Tyr
            20

<210>  26
<211>  10
<212>  PRT
<213>  Homo sapiens

<400>  26

Lys Ile Leu Gln Gln Ser Arg Val Val Gln
1               5                   10

<210>  27
<211>  11
<212>  PRT
<213>  Homo sapiens

<400>  27

Lys Ile Leu Gln Gln Ser Arg Val Val Gln Ser
1               5                   10

<210>  28
<211>  12
<212>  PRT
<213>  Homo sapiens

<400>  28

Lys Ile Leu Gln Gln Ser Arg Val Val Gln Ser Gln
1               5                   10

<210>  29
<211>  12
<212>  PRT
<213>  Homo sapiens

<400>  29

Gln Lys Ile Leu Gln Gln Ser Arg Val Val Gln Ser
1               5                   10

<210>  30
<211>  12
<212>  PRT
<213>  Homo sapiens

<400>  30

Gln Gln Lys Ile Leu Gln Gln Ser Arg Val Val Gln
1               5                   10

<210>  31
<211>  19
<212>  PRT
<213>  Homo sapiens

<400>  31

Gln Gln Gln Lys Ile Leu Gln Gln Ser Arg Val Val Gln Ser Gln Arg
1               5                   10                  15

Leu Lys Thr

<210>  32
<211>  20
<212>  PRT
<213>  Homo sapiens

<400>  32

Arg Gln Gln Gln Lys Ile Leu Gln Gln Ser Arg Val Val Gln Ser Gln

1               5                   10                  15

Arg Leu Lys Thr
            20

<210>  33
<211>  24
<212>  PRT
<213>  Homo sapiens

<400>  33

Leu Asn Met Phe Arg Gln Gln Gln Lys Ile Leu Gln Gln Ser Arg Val
1               5                   10                  15

Val Gln Ser Gln Arg Leu Lys Thr
            20

<210>  34
<211>  20
<212>  PRT
<213>  Homo sapiens

<400>  34

Gln Gln Gln Lys Ile Leu Gln Gln Ser Arg Val Val Gln Ser Gln Arg
1               5                   10                  15

Leu Lys Thr Ile
            20

<210>  35
<211>  24
<212>  PRT
<213>  Homo sapiens

<400>  35

Gln Gln Gln Lys Ile Leu Gln Gln Ser Arg Val Val Gln Ser Gln Arg
1               5                   10                  15

Leu Lys Thr Ile Lys Gln Leu Tyr
            20